# EUROPEAN PATENT SPECIFICATION

(11) **EP 1 335 981 B1**
(45) Date of publication and mention of the grant of the patent: **09.06.2010**
(21) Application number: 01959043.9
(22) Date of filing: 18.07.2001
(51) Int. Cl.: C12N 15/54, C12N 9/12, C07K 16/40, C12Q 1/68, A61K 38/45, A61K 48/00, G01N 33/50, G01N 33/68

(54) **18480 HUMAN PROTEIN KINASE MOLECULES AND USES THEREFOR**
18480 HUMANE PROTEINKINASEMOLEKÜLE UND IHRE VERWENDUNGEN
NOUVELLES MOLECULES HUMAINES DE PROTEINES KINASES 18480 ET UTILISATIONS CORRESPONDANTES

(30) Priority: 18.07.2000 US 219028 P
(43) Date of publication of application: 20.08.2003
(73) Proprietor: MILLENNIUM PHARMACEUTICALS, INC., Cambridge, MA 02139 (US)
(72) Inventor: MEYERS, Rachel, Newton, MA 02468 (US); KAPELLER-LIBERMANN, Rosana, Chestnut Hill, MA 02467 (US); RUDOLPH-OWEN, Laura, Jamaica Plain, MA 02130 (US); TSAI, Fong-Ying, Newton, MA 02468 (US)
(74) Representative: Taylor, Kate Laura
(86) International application number: PCT/US2001/022820
(87) International publication number: WO 2002/006330

(56) References cited:
- WO-A-00/22143
- WO-A-02/42438
- WO-A-97/33909
- WO-A-99/34015
- WO-A-02/098917
- WO-A-02/099096
- DATABASE EMBL [Online] 24 May 2000 (2000-05-24), "RC1-CT0199-180999-012-F03 CT0199 Homo sapiens cDNA, mRNA sequence." XP002371700 retrieved from EBI accession no. EM_PRO:AW847022 Database accession no. AW847022
- DATABASE EMBL [Online] 6 February 2000 (2000-02-06), "QV4-ST0234-121199-032-c04 ST0234 Homo sapiens cDNA, mRNA sequence." XP002371701 retrieved from EBI accession no. EM_PRO:AW391942 Database accession no. AW391942
- ZHANG H ET AL.: "Cloning, characterization, and chromosome mapping of RPS6KC1, a novel putative member of the ribosome protein S6 kinase family, to chromosome 12q12-q13.1." GENOMICS, vol. 61, no. 3, 1 November 1999 (1999-11-01), pages 314-318, XP002199848 ISSN: 0888-7543
- DATABASE WPI Section Ch, Week 200050 Derwent Publications Ltd., London, GB; Class B04, AN 2000-544302 XP002199851 & CN 1 257 929 A (UNIV FUDAN), 28 June 2000 (2000-06-28)
- DUFNER A ET AL.: "Ribosomal S6 kinase signaling and the control of translation." EXPERIMENTAL CELL RESEARCH, vol. 253, no. 1, 25 November 1999 (1999-11-25), pages 100-109, XP002199849 ISSN: 0014-4827
- FRODIN M ET AL.: "Role and regulation of 90 kDa ribosomal S6 kinase (RSK) in signal transduction." MOLECULAR AND CELLULAR ENDOCRINOLOGY, vol. 151, no. 1-2, 25 May 1999 (1999-05-25), pages 65-77, XP001077704 ISSN: 0303-7207
- TOKER A: "Protein kinases as mediators of phosphoinositide 3-kinase signaling." MOLECULAR PHARMACOLOGY, vol. 57, no. 4, April 2000 (2000-04), pages 652-658, XP002199850 ISSN: 0026-895X

## Description

### Background of the Invention

Phosphate tightly associated with protein has been known since the late nineteenth century. Since then, a variety of covalent linkages of phosphate to proteins have been found. The most common involve esterification of phosphate to serine, threonine, and tyrosine with smaller amounts being linked to lysine, arginine, histidine, aspartic acid, glutamic acid, and cysteine. The occurrence of phosphorylated proteins implies the existence of one or more protein kinase capable of phosphorylating amino acid residues on proteins, and also of protein phosphatases capable of hydrolyzing phosphorylated amino acid residues on proteins.

Kinases play a critical role in the mechanism of intracellular signal transduction. They act on the hydroxyamino acids of target proteins to catalyze the transfer of a high energy phosphate group from adenosine triphosphate (ATP). This process is known as protein phosphorylation. Along with phosphatases, which remove phosphates from phosphorylated proteins, kinases participate in reversible protein phosphorylation. Reversible phosphorylation acts as the main strategy for regulating protein activity in eukaryotic cells.

Protein kinases play critical roles in the regulation of biochemical and morphological changes associated with cell proliferation, differentiation, growth and division (D'Urso, G. et al. (1990) Science 250: 786-791; Birchmeier. C. et al. (1993) Bioessays 15: 185-189). They serve as growth factor receptors and signal transducers and have been implicated in cellular transformation and malignancy (Hunter, T. et al. (1992) Cell 70: 375-387; Posada, J. et al. (1992) Mol.Biol. Cell 3: 583-592; Hunter, T. et al. (1994) Cell 79: 573-582). For example, protein kinases have been shown to participate in the transmission of signals from growth-factor receptors (Sturgill, T. W. et al. (1988) Nature 344: 715-718; Gomez, N. et al. (1991) Nature 353: 170-173), control of entry of cells into mitosis (Nurse, P. (1990) Nature 344: 503-508; Maller, J. L. (1991) Curr. Opin. Cell Biol. 3: 269-275) and regulation of actin bundling (Husain-Chishti, A. et al. (1988) Nature 334: 718-721).

Kinases vary widely in their selectivity and specificity of target proteins. They still may, however, comprise the largest known enzyme superfamily. Protein kinases can be divided into two main groups based on either amino acid sequence similarity or specificity for either serine/threonine or tyrosine residues. Serine/threonine specific kinases are often referred to as STKs while tyrosine specific kinases are referred to as PTKs. A small number of dual-specificity kinases are structurally like the serine/threonine-specific group. Within the broad classification, kinases can be further sub-divided into families whose members share a higher degree of catalytic domain amino acid sequence identity and also have similar biochemical properties. Most protein kinase family members also share structural features outside the kinase domain that reflect their particular cellular roles. These include regulatory domains that control kinase activity or interaction with other proteins (Hanks, S.K. et al. (1988) Science 241: 42-52).

Almost all kinases contain a catalytic domain composed of 250-300 conserved amino acids. This catalytic domain may be viewed as composed of 11 subdomains. Some of these subdomains apparently contain distinct amino acid motifs which confer specificity as a STK or PTK or both. Kinases may also contain additional amino acid sequences, usually between 5 and 100 residues, flanking or occurring within the catalytic domain. These residues apparently act to regulate kinase activity and to determine substrate specificity. (Reviewed in Hardie, G. and Hanks, S. (1995) The Protein Kinase Facts Book, Vol I:7-20 Academic Press, San Diego, Calif.)

Approximately one third of the known oncogenes encode PTKs. PTKs may occur as either transmembrane or soluble proteins. Transmembrane PTKs act as receptors for many growth factors. Interaction of a growth factor to its cognate receptor initiates the phosphorylation of specific tyrosine residues in the receptor itself as well as in certain second messenger proteins. Growth factors found to associate with such PTK receptors include epidermal growth factor, platelet-derived growth factor, fibroblast growth factor, hepatocyte growth factor, insulin and insulin-like growth factors, nerve growth factor, vascular endothelial growth factor, and macrophage colony stimulating factor.

Soluble PTKs often interact with the cytosolic domains of plasma membrane receptors. Receptors that signal through such PTKs include cytokine, hormone, and antigen-specific lymphocytic receptors. Many PTKs were identified as oncogene products by the observation that PTK activation was no longer subject to normal cellular controls. Also, increased tyrosine phosphorylation activity is often observed in cellular transformation, or oncogenesis, (Carbonneau, H. and Tonks, N. K. (1992) Annu. Rev. Cell Biol.8:463-93.) PTK regulation may therefore be an important strategy in controlling some types of cancer.

### Summary of the Invention

The present invention is based, at least in part, on the discovery of novel nucleic acid molecules and proteins encoded by such nucleic acid molecules, referred to herein as "kinases" or by the clone name "18480". The 18480 nucleic acid and protein molecules of the present invention are useful as modulating agents in regulating a variety of cellular processes, e.g., including cell proliferation, differentiation, growth and division. In particular, the kinases and their related nucleic acids will be advantageous in the regulation of any cellular function, uncontrolled proliferation and differentiation, such as in cases of cancer. Other situations where the kinases of the invention are of particular advantage are in cases of autoimmune disorders or undesired inflammation. Accordingly, in one aspect, this invention provides isolated nucleic acid molecules encoding 18480 proteins or biologically active portions thereof, as well as nucleic acid fragments suitable as primers or hybridization probes for the detection of 18480-encoding nucleic acids.

The nucleotide sequence of cDNA encoding 18480 is shown in SEQ ID NO:4, and the amino acid sequence of a 18480 polypeptide is shown in SEQ ID NO:5. In addition, the nucleotide sequence of the coding region is depicted in SEQ ID NO:6.

Accordingly, in one aspect, the invention features nucleic acid molecules which encode a 18480 protein or polypeptide, e.g., a biologically active portion of the 18480 protein. In a preferred embodiment, the isolated nucleic acid molecule encodes a polypeptide having the amino acid sequence of SEQ ID NO:5. In other embodiments, the invention provides an isolated 18480 nucleic acid molecule having the nucleotide sequence shown in SEQ ID NO:4 or SEQ ID NO:6. In still other embodiments, the invention provides nucleic acid molecules that are substantially identical (e.g., naturally occurring allelic variants) to the nucleotide sequence shown in SEQ ID NO:4, or SEQ ID NO:6 In other embodiments, the invention provides a nucleic acid molecule which hybridizes under stringent hybridization conditions to a nucleic acid molecule comprising the nucleotide sequence of SEQ ID NO:4 or SEQ ID NO:6,
wherein the nucleic acid encodes a full length 18480, protein or an active fragment thereof.

In a related aspect, the invention further provides nucleic acid constructs which include the 18480 nucleic acid molecules described herein. In certain embodiments, the nucleic acid molecules of the invention are operatively linked to native or heterologous regulatory sequences. Also included, are vectors and host cells containing the 18480 nucleic acid molecules of the invention e.g., vectors and host cells suitable for producing 18480 nucleic acid molecules and polypeptides.

In another related aspect, the invention provides nucleic acid fragments suitable as primers or hybridization probes for the detection of 18480-encoding nucleic acids.

In still another related aspect, isolated nucleic acid molecules that are antisense to 18480 encoding nucleic acid molecules are provided.

In another aspect, the invention features 18480 polypeptides, and biologically active or antigenic fragments thereof that are useful, e.g., as reagents or targets in assays applicable to treatment and diagnosis of 18480-mediated or -related disorders. In another embodiment, the invention provides 18480 polypeptides having a 18480 activity. Preferred polypeptides are 18480 proteins including at least one protein kinase domain, and preferably having a 18480 activity, e.g., a 18480, activity as described herein.

In other embodiments, the invention provides 18480 polypeptides, e.g., a 18480 polypeptide having the amino acid sequence shown in SEQ ID NO:2, 5; amino acid sequences that are substantially identical to the amino acid sequences shown in SEQ ID NO: 5; or an amino acid sequence encoded by a nucleic acid molecule having a nucleotide sequence which hybridizes under stringent hybridization conditions to a nucleic acid molecule comprising the nucleotide sequences of SEQ ID NO:4 or SEQ ID NO:6, wherein the nucleic acid encodes a full length 18480 protein or an active fragment thereof.

In a related aspect, the invention further provides nucleic acid constructs which include the 18480 nucleic acid molecules described herein.

In a related aspect, the invention provides 18480 polypeptides or fragments operatively linked to non-18480 polypeptides to form fusion proteins.

In another aspect, the invention features antibodies and antigen-binding fragments thereof, that react with, or more preferably specifically bind 18480 polypeptides.

In another aspect, the invention provides methods of screening for compounds that modulate the expression or activity of the 18480 polypeptides or nucleic acids.

In still another aspect, the invention provides a process for modulating 18480 polypeptides or nucleic acid expression or activity, e.g. using the screened compounds. In certain embodiments, the methods involve treatment of conditions related to aberrant activity or expression of the 18480 polypeptides or nucleic acids, such as conditions involving aberrant or deficient cellular proliferation or differentiation.

The invention also provides assays for determining the activity of or the presence or absence of 18480 polypeptides or nucleic acid molecules in a biological sample, including for disease diagnosis.

In further aspect the invention provides assays for determining the presence or absence of a genetic alteration in a 18480 polypeptide or nucleic acid molecule, including for disease diagnosis.

### Brief Description of the Drawings

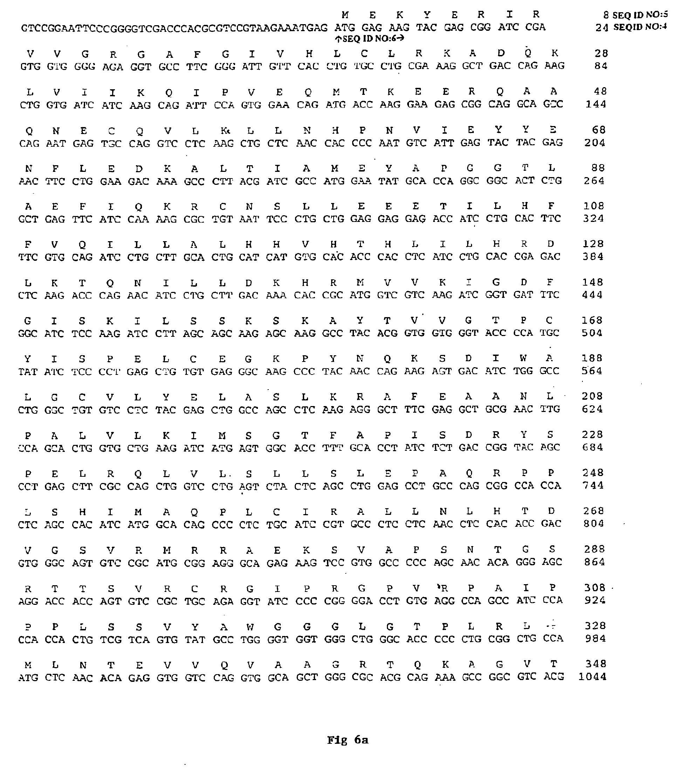

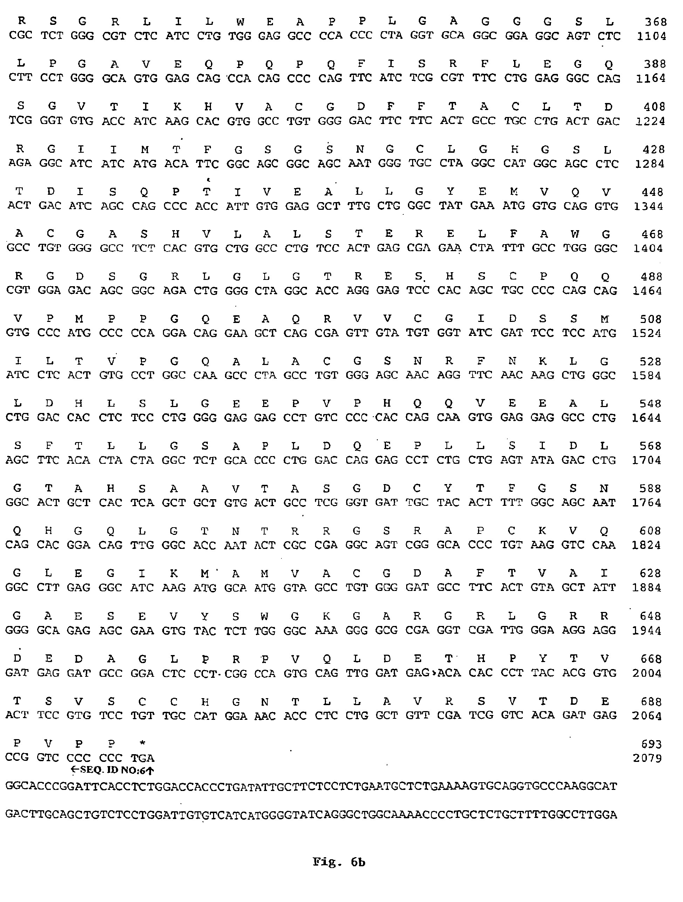

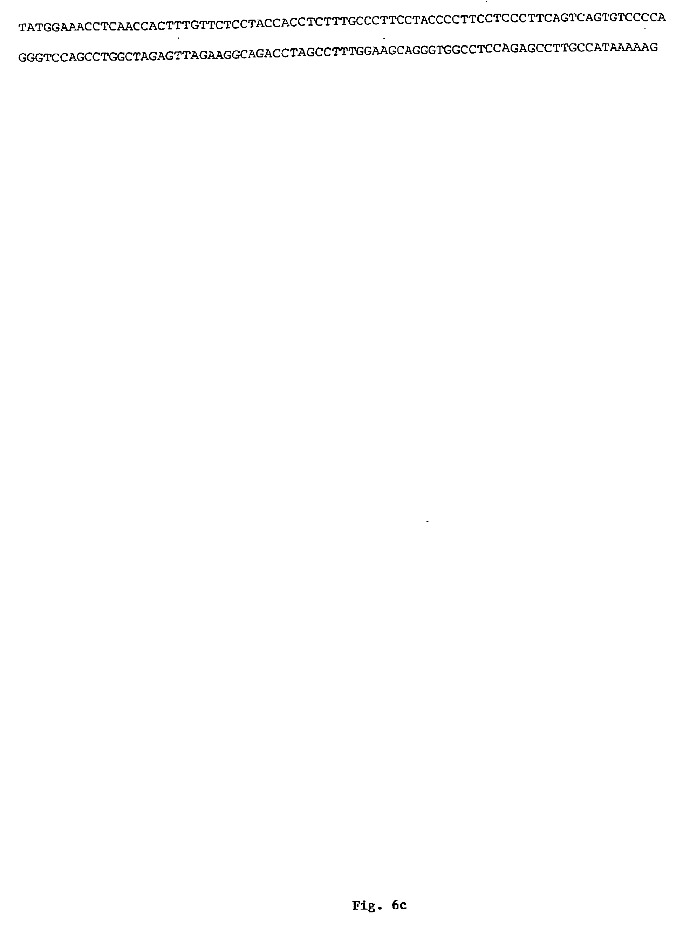

*Figures 6A-C* depict a cDNA sequence (SEQ ID NO:4) and predicted amino acid sequence (SEQ ID NO:5) of human 18480. The methionine-initiated open reading frame of human 18480 (without the 5' and 3' untranslated regions) extends from nucleotide position 1 to position 2079 of SEQ ID NO:6, not including the terminal codon.

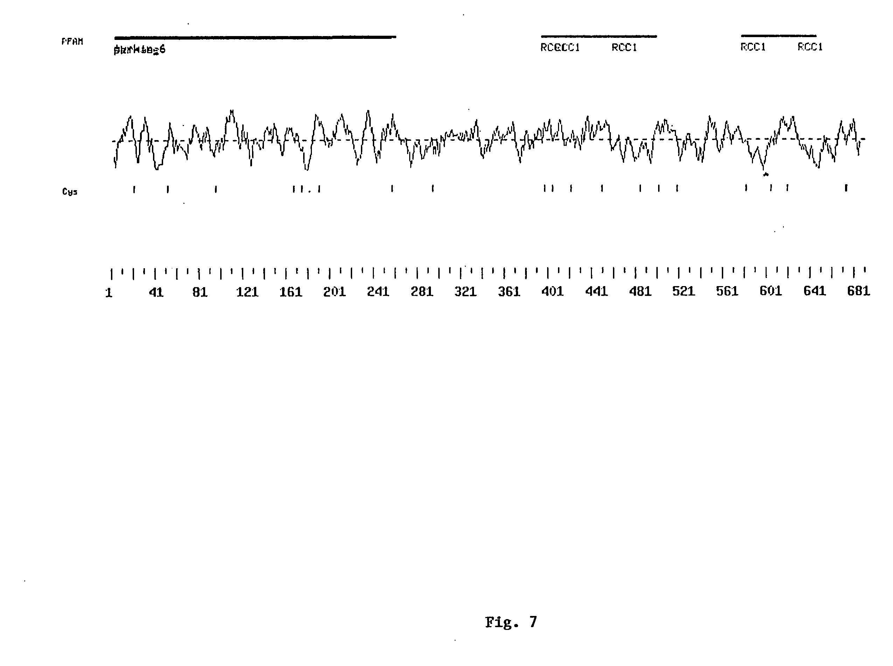

*Figure 7* depicts a hydropathy plot of human 18480. Relatively hydrophobic residues are shown above the dashed horizontal line, and relatively hydrophilic residues are below the dashed horizontal line. The cysteine residues (cys) are indicated by short vertical lines just below the hydropathy trace. The numbers corresponding to the amino acid sequence of human 18480 are indicated. Polypeptides of the invention include fragments which include: all or part of a hydrophobic sequence, e.g., a sequence above the dashed line, e.g., the sequence from about amino acid 10 to 20, from about 95 to 120, and from about 500 to 520 of SEQ ID NO:2; all or part of a hydrophilic sequence, e.g., a sequence below the dashed line, e.g., the sequence from about amino acid 40 to 55, from about 475 to 495, and from about 590 to 600 of SEQ ID NO:5; a sequence which includes a Cys, or a glycosylation site.

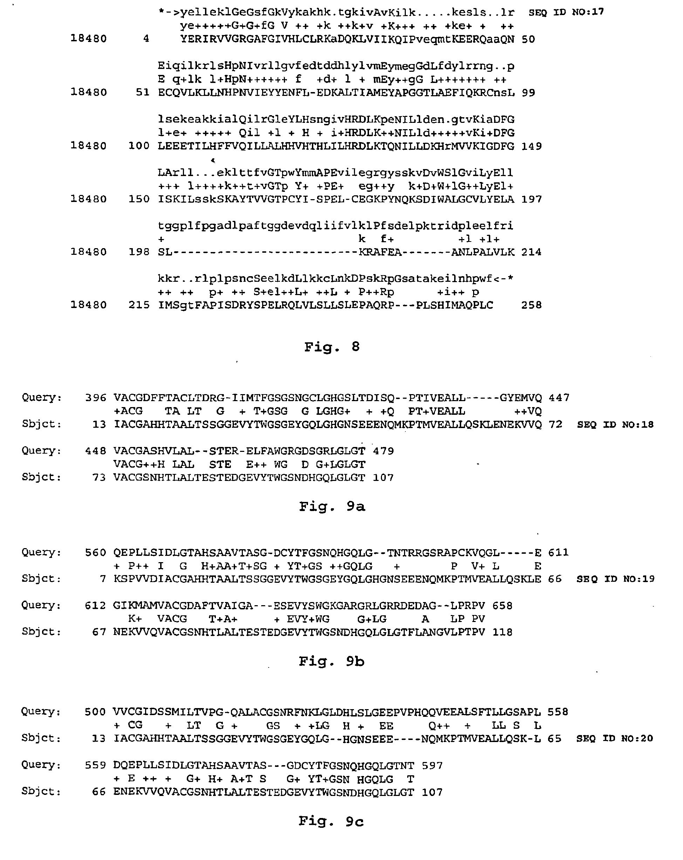

*Figure 8* depicts an alignment of the protein kinase domain of human 18480 with a consensus amino acid sequence derived from a hidden Markov model (HMM) from PFAM. The upper sequences are the consensus amino acid sequence (SEQ ID NO:17), while the lower amino acid sequences correspond to amino acids 4 to 258 of SEQ ID NO:5.

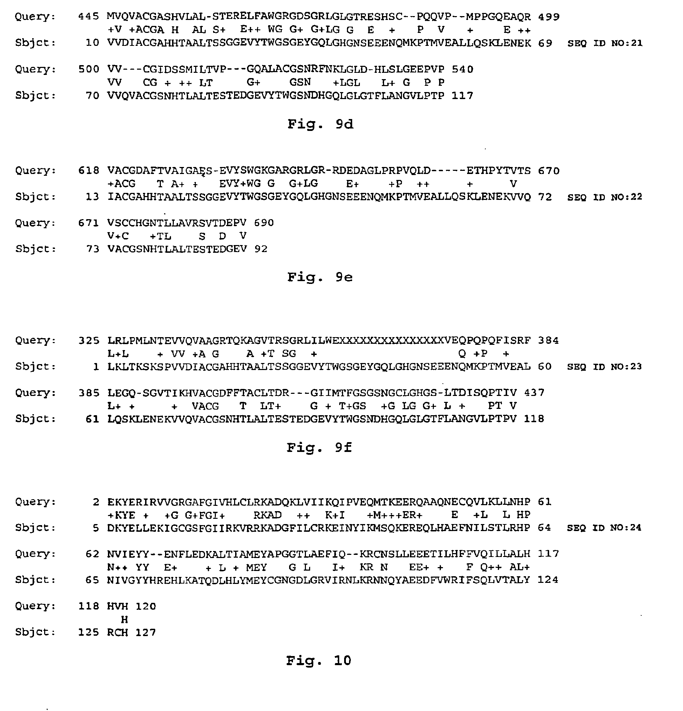

*Figures 9a**-f* depict a BLAST alignment of human 18480 with a consensus amino acid sequence derived from a ProDomain "regulator factor chromosome codensation repeat of guanine-nucleotide releasing cell cycle" (Release 2001.1; http://www.toulouse.inra.fr/prodom.html). The lower sequence is amino acid residues 325 to 690 of the amino acid consensus sequence (SEQ ID NOs:18-23), while the upper amino acid sequence corresponds to the "regulator factor chromosome codensation repeat of guanine-nucleotide releasing cell cycle" domain of human 18480, amino acid residues 396 to 479, 560 to 658, 500 to 597, 445 to 540, 618 to 690 and 325 to 437 of SEQ ID NO:5. The BLAST algorithm identifies multiple local alignments between the consensus amino acid sequence and human 18480. Figure 9A depicts the first local alignment, Figure 9B the second, Figure 9C the third, Figure 9D the fourth, Figure 9E the fifth, and Figure 9F the sixth.
*Figure 10* depicts a BLAST alignment of human 18480 with a consensus amino acid sequence derived from a ProDomain "kinase cell mitosis serine/threonine-protein cycle 2.7.1.- phosphorylation division nuclear G2-specific" (Release 2001.1; http://www.toulouse.inra.fr/prodom.html). The lower sequence is amino acid residues 5 to 127 of the 123 amino acid consensus sequence (SEQ ID NO:24), while the upper amino acid sequence corresponds to the "kinase cell mitosis serine/threonine-protein cycle 2.7.1.-phosphorylation division nuclear G2-specific" domain of human 18480, amino acid residues 2 to 120 of SEQ ID NO: 5.

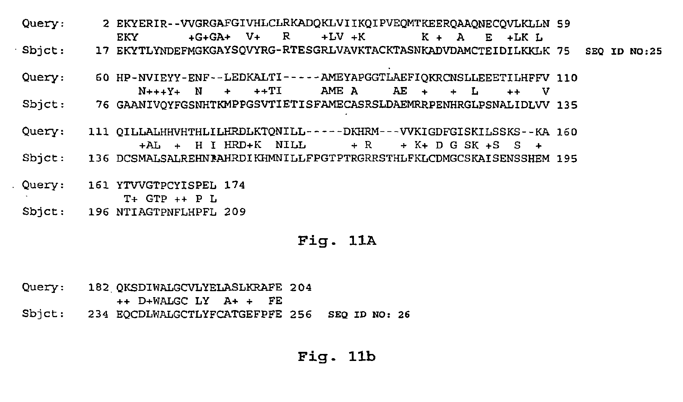

*Figures 11a**-b* depict a BLAST alignment of human 18480 with a consensus amino acid sequence derived from a ProDomain "kinase serine/threonine-protein Y39G8B.5 III R107.4 chromosome ATP-binding transferase 2.7.1" (Release 2001.1; http://www.toulouse.inra.fr/prodom.html). The lower sequence is amino acid residues 17 to 256 of the amino acid consensus sequence (SEQ ID NOs:25-26), while the upper amino acid sequence corresponds to the "kinase serine/threonine-protein Y39G8B.5 III R107.4 chromosome ATP-binding transferase 2.7.1" domain of human 18480, amino acid residues 2 to 174 and 182 to 204 of SEQ ID NO:5. The BLAST algorithm identifies multiple local alignments between the consensus amino acid sequence and human 18480. Figure 11A depicts the first local alignment and Figure 11B the second.

Other features and advantages of the invention will be apparent from the following detailed description, and from the claims.

### Detailed Description

### Human 18480

The human 18480 sequence (Figure 6A-C; SEQ ID NO:4), which is approximately 2438 nucleotides long including untranslated regions, contains a predicted methionine-initiated coding sequence of about 2079 nucleotides (nucleotides 45-2123 of SEQ ID NO:4; nucleotides 1-2079 of SEQ ID NO:6), not including the terminal codon. The coding sequence encodes a 692 amino acid protein (SEQ ID NO:5).

This mature protein form is approximately 692 amino acid residues in length (from about amino acid 1 to amino acid 692 of SEQ ID NO:5). The 18480 protein includes the following domains:
one predicted protein kinase domain (PFAM Accession Number PF00069) located at about amino acid residues 4 to 258 of SEQ ID NO:5;
one cAMP-dependent protein kinase phosphorylation site (PS00004) located at about amino acids 598-601 of SEQ ID NO:5;
thirteen predicted protein kinase C phosphorylation sites (PS00005) located at about amino acids 155-157, 198-200,224-226,271-273,292-294,342-344, 350-352, 392-394, 407-409, 460-462, 472-474, 521-523 and 597-599 of SEQ ID NO:5;
eight predicted casein kinase II phosphorylation sites (PS00006) located at about amino 41-44, 87-90, 98-101, 427-430, 435-438, 460-463, 533-536 and 684-687 of SEQ ID NO:5;
thirteen predicted N-myristoylation sites (PS00008) located at about amino acids 85-90, 287-292, 318-323, 346-351, 362-367, 410-415, 416-421, 478-483, 503-508, 514-519, 569-574, 591-596 and 612-617 of SEQ ID NO:5;
one predicted amidation site (PS00009) located at about amino acids 645-648 of SEQ ID NO:5;
one predicted prokaryotic membrane lipoprotein lipid attachment site (PS00013) located at about amino acids 663-673 of SEQ ID NO:5;
one cell attachment site (PS00016) located at about amino acids 469-471 of SEQ ID NO:5;
one ATP-binding region signature site (PS00107) located at about amino acids 10-18 of SEQ ID NO:5; and
one serine/threonine kinase active site signature located at about amino acids 124-136 of SEQ ID NO:5.

In one embodiment, a 18480 family member can include at least one protein kinase domain (PFAM Accession Number PF00069). Furthermore, a 18480 family member can include at least one cAMP-dependent protein kinase phosphorylation site (PS00004); at least one, two, three, four, five, six, seven, eight, nine, ten, eleven, twelve, and preferably thirteen protein kinase C phosphorylation sites (PS00005); at least one, two, three, four, five, six, seven, and preferably eight casein kinase II phosphorylation sites (PS00006); at least one, two, three, four, five, six, seven, eight, nine, ten, eleven, twelve, and preferably thirteen N-myristolyation sites (PS00008); at least one amidation site (PS00009); at least one prokaryotic membrane lipoprotein lipid attachment site (PS00013); at least one cell attachment site (PS00016); at least one ATP-binding region signature site (PS00107); at least one serine/threonine kinase active site signature

In another embodiment, the isolated proteins of the present invention, preferably 18480 proteins, are identified based on the presence of at least one Ser/Thr kinase site and at least one ATP-binding region.

As used herein, the term "ATP-binding region" includes an amino acid sequence of about 20-40, preferably 20-30, and more preferably 25-30 amino acid residues in length, present in enzymes which activate their substrates by phosphorylation, and involved in binding adenosine triphosphate (ATP). ATP-binding regions preferably include the following amino acid consensus sequence: G-X-G-X-X-G-X(15-23)-K [SEQ ID NO:38]. ATP-binding regions are described in, for example, Samuel K.P. et al. (1987) FEBS Let. 218(1): 81-86, the contents of which are incorporated herein by reference. Amino acid residues 10 to 18 of comprise an ATP-binding region. Amino acid residues 124 to 136 of the 18480 protein comprise a Ser/Thr kinase domain.

Accordingly, another embodiment of the invention features isolated 18480 proteins and polypeptides having a 18480 activity. Preferred proteins are 18480 proteins having at least one Ser/Thr kinase and at least one ATP-binding region. Additional preferred proteins have at least one Ser/Thr kinase site, at least one ATP-binding region, and preferably a 18480 activity. Additional preferred proteins have at least one Ser/Thr kinase site, at least one ATP-binding region, and are, preferably, encoded by a nucleic acid molecule having a nucleotide sequence which hybridizes under stringent hybridization conditions to a nucleic acid molecule comprising the nucleotide sequence of SEQ ID NO:4 or SEQ ID NO:6.

The nucleic acid encodes a polypeptide with similarities known Ser/Thr kinases. Thus the 18480 encoded polypeptide is expected to be a kinase and function in the phosphorylation of protein substrates. Additionally, the 18480 nucleic acids can be used in known or novel screens and assays for kinase encoding nucleic acids to distinguish it from other distinct nucleic acids. Alternatively, the nucleic acid sequences can be used in the preparation of phylogenetic trees and relationships between organisms.

The 18480 proteins contain a significant number of structural characteristics in common with members of the protein kinase family. The term "family" when referring to the protein and nucleic acid molecules of the invention means two or more proteins or nucleic acid molecules having a common structural domain or motif and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., rat or mouse proteins. Members of a family can also have common functional characteristics.

As used herein, the term "protein kinase" includes a protein or polypeptide which is capable of modulating its own phosphorylation state or the phosphorylation state of another protein or polypeptide. Protein kinases can have a specificity for (i.e., a specificity to phosphorylate) serine/threonine residues, tyrosine residues, or both serine/threonine and tyrosine residues, e.g., the dual specificity kinases. As referred to herein, protein kinases preferably include a catalytic domain of about 200-400 amino acid residues in length, preferably about 200-300 amino acid residues in length, or more preferably about 250-300 amino acid residues in length, which includes preferably 5-20, more preferably 5-15, or preferably 11 highly conserved motifs or subdomains separated by sequences of amino acids with reduced or minimal conservation. Specificity of a protein kinase for phosphorylation of either tyrosine or serine/threonine can be predicted by the sequence of two of the subdomains (VIb and VIII) in which different residues are conserved in each class (as described in, for example, Hanks et al. (1988) Science 241:42-52) the contents of which are incorporated herein by reference). These subdomains are also described in further detail herein.

Protein kinases play a role in signaling pathways associated with cellular growth. For example, protein kinases are involved in the regulation of signal transmission from cellular receptors, e.g., growth-factor receptors; entry of cells into mitosis; and the regulation of cytoskeleton function, e.g., actin bundling. Thus, the 18480 molecules of the present invention may be involved in: 1) the regulation of transmission of signals from cellular receptors, e.g., cell growth factor receptors; 2) the modulation of the entry of cells into mitosis; 3) the modulation of cellular differentiation; 4) the modulation of cell death; and 5) the regulation of cytoskeleton function, e.g., actin bundling.

Inhibition or over stimulation of the activity of protein kinases involved in signaling pathways associated with cellular growth can lead to perturbed cellular growth, which can in turn lead to cellular growth related disorders. As used herein, a "cellular growth related disorder" includes a disorder, disease, or condition characterized by a deregulation, e.g., an upregulation or a downregulation, of cellular growth. Cellular growth deregulation may be due to a deregulation of cellular proliferation, cell cycle progression, cellular differentiation and/or cellular hypertrophy.

A 18480 polypeptide can include a "kinase domain" or regions homologous with an "kinase domain".

As used herein, the term "kinase domain" includes an amino acid sequence of about 10-500 amino acid residues in length and having a bit score for the alignment of the sequence to the kinase domain (HMM) of at least 8.Preferably, a kinase domain includes at least about 20-350 amino acids, more preferably about 25-325 amino acid residues, or about 30-310 amino acids and has a bit score for the alignment of the sequence to the kinase domain (HMM) of at least 16 or greater. The kinase domain (HMM) has been assigned the PFAM Accession PF01553 (http://pfam.wustl.edu/). An alignment of the kinase domain of human 18480 with a consensus amino acid sequence derived from a hidden Markov model is depicted in Figures 3, 8,14, and 18.

In a preferred embodiment 18480 polypeptide or protein has a "kinase domain" or a region which includes at least about 10-500 more preferably about 20-350 or 30-310 amino acid residues and has at least about 60%, 70%, 80%, 90%, 95%, 99%, or 100% homology with an "kinase domain," e.g., the kinase domain of human

18480.

To identify the presence of an "kinase" domain in a 18480 protein sequence, and make the determination that a polypeptide or protein of interest has a particular profile, the amino acid sequence of the protein can be searched against a database of HMMs (e.g., the Pfam database, release 2.1) using the default parameters (http://www.sanger.ac.uk/Software/Pfam/HMM_search). For example, the hmmsf program, which is available as part of the HMMER package of search programs, is a family specific default program for MILPAT0063 and a score of 15 is the default threshold score for determining a hit. Alternatively, the threshold score for determining a hit can be lowered (e.g., to 8 bits). A description of the Pfam database can be found in Sonhammer et al., (1997) Proteins 28(3):405-420 and a detailed description of HMMs can be found, for example, in Gribskov et al., (1990) Meth. Enzymol. 183:146-159; Gribskov et al., (1987) Proc. Natl. Acad. Sci. USA 84:4355-4358; Krogh et al., (1994) J. Mol. Biol. 235:1501-1531; and Stultz et al., (1993) Protein Sci. 2:305-314, the contents of which are incorporated herein by reference. A search was performed against the HMM database resulting in the identification of a "kinase" domain in the amino acid sequence of human 18480 at about residues 4 to 258 of SEQ ID NO:5 (see Figure 8).

For further identification of domains in a 18480 protein sequence, and make the determination that a polypeptide or protein of interest has a particular profile, the amino acid sequence of the protein can be searched against a database of domains, e.g., the ProDom database (Corpet et al. (1999), Nucl. Acids Res. 27:263-267). The ProDom protein domain database consists of an automatic compilation of homologous domains. Current versions of ProDom are built using recursive PSI-BLAST searches (Altschul SF et al.(1997) Nucleic Acids Res. 25:3389-3402*;* Gouzy et al. (1999) 23:333-340) of the SWISS-PROT 38 and TREMBL protein databases. The database automatically generates a consensus sequence for each domain. A BLAST search was performed against the HMM database resulting in the identification of a "kinase" domain(s) in the amino acid sequence of human 18480 at about residues 396 to 479, 560 to 658, 500 to 597, 445 to 540, 618 to 690, and 325 to 437 (six local alignments) of SEQ ID NO:5 (see Figure 9) having 50%,36%, 34%, 35%, 30% and 27% identity over those residues respectively; 2 to 120 of SEQ ID NO:5 (see Figure 10) having 34% identity over those residues; 2 to 174 and 182 to 204 of SEQ ID NO:5 (two local alignments) having 28% and 47% identity over those residues respectively.

As the 18480 polypeptides of the invention may modulate 18480- -mediated activities, they may be useful for developing novel diagnostic and therapeutic agents for 18480-, -mediated or related disorders, as described below.

As used herein, a "18480 activity", "biological activity of 18480" or "functional activity of 18480", refers to an activity exerted by a 18480 protein, polypeptide or nucleic acid molecule on e.g., a 18480- -responsive cell or on a 18480 substrate, e.g., a lipid or protein substrate, as determined in vivo or *in vitro.* In one embodiment, a 18480 activity is a direct activity, such as an association with a 18480 target molecule. A "target molecule" or "binding partner" is a molecule with which a 18480 protein binds or interacts in nature, e.g., a protein to which the 18480 protein attaches a phosphate. A 18480 activity can also be an indirect activity, e.g., a cellular signaling activity mediated by interaction of the 18480 protein with a 18480 ligand.

Accordingly, 18480 proteins may mediate various disorders, including cellular proliferative and/or differentiative disorders, brain disorders, heart disorders, blood vessel disorders, and platelet disorders.

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast, ovary and liver origin.

As used herein, the terms "cancer", "hyperproliferative" and "neoplastic" refer to cells having the capacity for autonomous growth, i.e., an abnormal state or condition characterized by rapidly proliferating cell growth. Hyperproliferative and neoplastic disease states may be categorized as pathologic, i.e., characterizing or constituting a disease state, or may be categorized as non-pathologic, i.e., a deviation from normal but not associated with a disease state. The term is meant to include all types of cancerous growths or oncogenic processes, metastatic tissues or malignantly transformed cells, tissues, or organs, irrespective of histopathologic type or stage of invasiveness. "Pathologic hyperproliferative" cells occur in disease states characterized by malignant tumor growth. Examples of non-pathologic hyperproliferative cells include proliferation of cells associated with wound repair.

The terms "cancer" or "neoplasms" include malignancies of the various organ systems, such as affecting lung, breast, thyroid, lymphoid, gastrointestinal, and genitourinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus.

The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures.

The term "sarcoma" is art recognized and refers to malignant tumors of mesenchymal derivation.

The 18480 nucleic acid and protein of the invention can be used to diagnose lung cancer or colon cancer

The 18480 proteins, fragments thereof, and derivatives and other variants of the sequences in SEQ ID NO:5, are collectively referred to as "polypeptides or proteins of the invention" or " 18480 polypeptides or proteins". Nucleic acid molecules encoding such polypeptides or proteins are collectively referred to as "nucleic acids of the invention" or "18480 nucleic acids." 18480 molecules refer to 18480 nucleic acids, polypeptides, and antibodies.

As used herein, the term "nucleic acid molecule" includes DNA molecules (e.g., a cDNA or genomic DNA) and RNA molecules (e.g., an mRNA) and analogs of the DNA or RNA generated, e.g., by the use of nucleotide analogs. The nucleic acid molecule can be single-stranded or double-stranded, but preferably is double-stranded DNA.

The term "isolated or purified nucleic acid molecule" includes nucleic acid molecules which are separated from other nucleic acid molecules which are present in the natural source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (i.e., sequences located at the 5' and/or 3' ends of the nucleic acid) in the genomic DNA of the organism from which the nucleic acid is derived. For example, in various embodiments, the isolated nucleic acid molecule can contain less than about 5 kb, 4kb, 3kb, 2kb, 1 kb, 0.5 kb or 0.1 kb of 5' and/or 3' nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under stringent conditions" describes conditions for hybridization and washing. Stringent conditions are known to those skilled in the art and can be found in Current Protocols in Molecular Biology, John Wiley & Sons, N.Y. (1989), 6.3.1-6.3.6. Aqueous and nonaqueous methods are described in that reference and either can be used. A preferred, example of stringent hybridization conditions are hybridization in 6X sodium chloride/sodium citrate (SSC) at about 45°C, followed by one or more washes in 0.2X SSC, 0.1% SDS at 50°C. Another example of stringent hybridization conditions are hybridization in 6X sodium chloride/sodium citrate (SSC) at about 45°C, followed by one or more washes in 0.2X SSC, 0.1% SDS at 55°C. A further example of stringent hybridization conditions are hybridization in 6X sodium chloride/sodium citrate (SSC) at about 45°C, followed by one or more washes in 0.2X SSC, 0.1% SDS at 60°C. Preferably, stringent hybridization conditions are hybridization in 6X sodium chloride/sodium citrate (SSC) at about 45°C, followed by one or more washes in 0.2X SSC, 0.1% SDS at 65°C. Particularly preferred stringency conditions, (and the conditions that should be used if the practitioner is uncertain about what conditions should be applied to determine if a molecule is within a hybridization limitation of the invention) are 0.5M Sodium Phosphate, 7% SDS at 65°C, followed by one or more washes at 0.2X SSC, 1 % SDS at 65°C. Preferably, an isolated nucleic acid molecule of the invention that hybridizes under stringent conditions to the sequence of SEQ ID NO:4, or SEQ ID NO:6 corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein).

As used herein, the terms "gene" and "recombinant gene" refer to nucleic acid molecules which include an open reading frame encoding a 18480 protein, preferably a mammalian 18480 protein, and can further include non-coding regulatory sequences, and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 18480 protein having less than about 30%, 20%, 10% and more preferably 5% (by dry weight), of non- -18480, protein (also referred to herein as a "contaminating protein"), or of chemical precursors or non- -18480 chemicals. When the 18480 protein or biologically active portion thereof is recombinantly produced, it is also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 18480 (e.g., the sequence of SEQ ID NO:4 or SEQ ID NO:6) without abolishing or more preferably, without substantially altering a biological activity, whereas an "essential" amino acid residue results in such a change. For example, amino acid residues that are conserved among the polypeptides of the present invention, e.g., those present in the protein kinase domain, are predicted to be particularly unamenable to alteration.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in a 18480 protein is preferably replaced with another amino acid residue from the same side chain family. Alternatively, in another embodiment, mutations can be introduced randomly along all or part of a 18480 coding sequence, such as by saturation mutagenesis, and the resultant mutants can be screened for

18480 biological activity to identify mutants that retain activity. Following mutagenesis of SEQ ID NO:4; or SEQ ID NO:6, the encoded protein can be expressed recombinantly and the activity of the protein can be determined.

As used herein, a "biologically active portion" of a 18480 protein includes a fragment of a 18480 protein which participates in an interaction between a 18480 molecule and a -18480, molecule. Biologically active portions of a 18480 protein include peptides comprising amino acid sequences sufficiently homologous to or derived from the amino acid sequence of the 18480 protein, e.g., the amino acid sequence shown in SEQ ID NO:5, which include less amino acids than the full length 18480 proteins, and exhibit at least one activity of a 18480 protein. Typically, biologically active portions comprise a domain or motif with at least one activity of the 18480 protein, e.g., protein kinase activity. A biologically active portion of a 18480 protein can be a polypeptide which is, for example, 10, 25, 50, 100, 200 or more amino acids in length. Biologically active portions of a 18480 protein can be used as targets for developing agents which modulate a 18480 mediated activity, e.g., protein kinase activity.

Calculations of homology or sequence identity between sequences (the terms are used interchangeably herein) are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence (e.g., when aligning a second sequence to the 13237 amino acid sequence of SEQ ID NO:2 having 1066 amino acid residues, at least 320, preferably at least 427, more preferably at least 534, even more preferably at least 640,and even more preferably at least 747, 854, 960 or 1067 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (J. Mol. Biol. (48):444-453 (1970)) algorithm which has been incorporated into the GAP program in the GCG software package (available at http://www.gcg.com), using either a Blossum 62 matrix or a PAM250 matrix, and a gap weight of 16,14,12,10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) is using a Blossum 62 scoring matrix with a gap open penalty of 12, a gap extend penalty of 4, and a frameshift gap penalty of 5.

The percent identity between two amino acid or nucleotide sequences can be determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performer using the NBLAST and XBLAST programs (version 2.0) of Altschul, et al., (1990) J. Mol. Biol. 215:403-10. BLAST nucleotide searches can be performed with the NBLAST program, score = 100, wordlength = 12 to obtain nucleotide sequences homologous to 13237, 18480, 2245 or 16228 nucleic acid molecules of the invention. BLAST protein searches can be performed with the XBLAST program, score = 50, wordlength = 3 to obtain amino acid sequences homologous to 13237, 18480, 2245 or 16228 protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used. See http://www.ncbi.nlm.nih.gov.

"Misexpression or aberrant expression", as used herein, refers to a non-wild type pattern of gene expression, at the RNA or protein level. It includes: expression at non-wild type levels, i.e., over or under expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject", as used herein, can refer to a mammal, e.g., a human, or to an experimental or animal or disease model. The subject can also be a non-human animal, e.g., a horse, cow, goat, or other domestic animal.

A "purified preparation of cells", as used herein, refers to, in the case of plant or animal cells, an in vitro preparation of cells and not an entire intact plant or animal. In the case of cultured cells or microbial cells, it consists of a preparation of at least 10% and more preferably 50% of the subject cells.

Various aspects of the invention are described in further detail below.

### Isolated Nucleic Acid Molecules

In one aspect, the invention provides, an isolated or purified, nucleic acid molecule that encodes a 18480 polypeptide described herein, e.g., a full length 18480 protein or a fragment thereof, e.g., a biologically active portion of 18480 protein. Also included is a nucleic acid fragment suitable for use as a hybridization probe, which can be used, e.g., to a identify nucleic acid molecule encoding a polypeptide of the invention, 18480 mRNA, and fragments suitable for use as primers, e.g., PCR primers for the amplification or mutation of nucleic acid molecules.

In one embodiment, an isolated nucleic acid molecule of the invention includes the nucleotide sequence shown in SEQ ID NO:4 or a portion of any of these nucleotide sequences. In one embodiment, the nucleic acid molecule includes sequences encoding the human 18480 protein (i.e., "the coding region", from nucleotides 76-3277, 45-2123,1-1278 or 36-3017 of SEQ ID NO:4, not including the terminal codon), as well as 5' untranslated sequences (nucleotides 75, 1-44 or 1-35 of SEQ ID NO:4 ). Alternatively, the nucleic acid molecule can include only the coding region of SEQ ID NO:4, (e.g., nucleotides 76-3277, 45-2123, 1-1278 or 36-3017 of SEQ ID NO:4 corresponding to or SEQ ID NO:6) and, e.g., no flanking sequences which normally accompany the subject sequence. In another embodiment, the nucleic acid molecule encodes a sequence corresponding to the mature protein of SEQ ID NO:5.

In another embodiment, an isolated nucleic acid molecule of the invention includes a nucleic acid molecule which is a complement of the nucleotide sequence shown in SEQ ID NO:4 or SEQ ID NO:6, or a portion of any of these nucleotide sequences. In other embodiments, the nucleic acid molecule of the invention is sufficiently complementary to the nucleotide sequence shown in SEQ ID NO:4 or SEQ ID NO:6 such that it can hybridize to the nucleotide sequence shown in SEQ ID NO:4, or SEQ ID NO:6, thereby forming a stable duplex.

In one embodiment, an isolated nucleic acid molecule of the present invention includes a nucleotide sequence which is at least about 60%, 65%, 70%, 75%, 80%, 85%, 90%, 91%, 92%, 93%, 94%, 95%, 96%, 97%, 98%, 99%, or more homologous to the nucleotide sequence shown in SEQ ID NO:4, or SEQ ID NO:6. In the case of an isolated nucleic acid molecule which is longer than or equivalent in length to the reference sequence, e.g., SEQ ID NO:4 or SEQ ID NO:6, the comparison is made with the full length of the reference sequence. Where the isolated nucleic acid molecule is shorter than the reference sequence, e.g., shorter than SEQ ID NO:4, or SEQ ID NO:6, the comparison is made to a segment of the reference sequence of the same length (excluding any loop required by the homology calculation).

### 18480 Nucleic Acid Fragments

A nucleic acid molecule of the invention can include only a portion of the nucleic acid sequence of SEQ ID NO:4, or SEQ ID NO: 6. For example, such a nucleic acid molecule can include a fragment which can be used as a probe or primer or a fragment encoding a portion of a 18480 protein, e.g., an immunogenic or biologically active portion of a 18480 protein. A fragment can comprise: nucleotides 12-774 or 7-786 of SEQ ID NO:6, which encodes an protein kinase domain of human 18480. The nucleotide sequence determined from the cloning of the 18480 gene allows for the generation of probes and primers designed for use in identifying and/or cloning other 18480 family members, or fragments thereof, as well as 18480 homologues, or fragments thereof, from other species.

In another embodiment, a nucleic acid includes a nucleotide sequence that includes part, or all, of the coding region and extends into either (or both) the 5' or 3' noncoding region. Other embodiments include a fragment which includes a nucleotide sequence encoding an amino acid fragment described herein. Nucleic acid fragments can encode a specific domain or site described herein or fragments thereof, particularly fragments thereof which are at least 150 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, the nucleic acid fragment can include an protein kinase domain. In a preferred embodiments the fragment is at least, 50, 100, 200, 300, 400, 500, 600, 700, or 900 base pairs in length.

18480 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30,35,40,45,50, 55,60,65, or 75 consecutive nucleotides of a sense or antisense sequence of SEQ ID NO:4 or SEQ ID NO:6, or of a naturally occurring allelic variant or mutant of SEQ ID NO:4, or SEQ ID NO:6.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes an protein kinase domain (e.g., about amino acid residues 4-258 of SEQ ID NO:5).

In another embodiment a set of primers is provided, e.g., primers suitable for use in a PCR, which can be used to amplify a selected region of a 18480 sequence, e.g., a region described herein. The primers should be at least 5, 10, or 50 base pairs in length and less than 100, or less than 200, base pairs in length. The primers should be identical, or differs by one base from a sequence disclosed herein or from a naturally occurring variant. E.g., primers suitable for amplifying all or a portion of any of the following regions are provided: an protein kinase domain (e.g., about amino acid residues 4-258 of SEQ ID NO:5).

A nucleic acid fragment can encode an epitope bearing region of a polypeptide described herein.

A nucleic acid fragment encoding a "biologically active portion of a 18480, polypeptide" can be prepared by isolating a portion of the nucleotide sequence of SEQ ID NO:4 or SEQ ID NO:6, which encodes a polypeptide having a 18480 biological activity (e.g., the biological activities of the 18480 proteins as described herein), expressing the encoded portion of the 18480 protein (e.g., by recombinant expression *in vitro*) and assessing the activity of the encoded portion of the 18480 protein. For example, a nucleic acid fragment encoding a biologically active portion of 18480 includes an protein kinase domain (e.g., about amino acid residues 4-258 of SEQ ID NO:5). A nucleic acid fragment encoding a biologically active portion of a 18480 polypeptide, may comprise a nucleotide sequence which is greater than 300-1200 or more nucleotides in length.

In preferred embodiments, nucleic acids include a nucleotide sequence which is about 300, 400, 500, 600, 700, 800, 900, 1000, 1100, 1200, 1300, 1400 nucleotides in length and hybridizes under stringent hybridization conditions to a nucleic acid molecule of SEQ ID NO:4, or SEQ ID NO:6.

### 13237, 18480, 2245 or 16228 Nucleic Acid Variants

The invention further encompasses nucleic acid molecules that differ from the nucleotide sequence shown in SEQ ID NO:4 or SEQ ID NO:6. Such differences can be due to degeneracy of the genetic code (and result in a nucleic acid which encodes the same 18480 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:5. If alignment is needed for this comparison the sequences should be aligned for maximum homology. "Looped" out sequences from deletions or insertions, or mismatches, are considered differences.

Nucleic acids of the inventor can be chosen for having codons, which are preferred, or non preferred, for a particular expression system. E.g., the nucleic acid can be one in which at least one colon, at preferably at least 10%, or 20% of the codons has been altered such that the sequence is optimized for expression in E. coli, yeast, human, insect, or CHO cells.

Nucleic acid variants can be naturally occurring, such as allelic variants (same locus), homologs (different locus), and orthologs (different organism) or can be non-naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO:4, or SEQ ID NO:6, e.g., as follows: by at least one but less than 10, 20, 30, or 40 nucleotides; at least one but less than 1%, 5%, 10% or 20% of the in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more identical to the amino acid sequence shown in SEQ ID NO:5 or a fragment of this sequence. Such nucleic acid molecules can readily be obtained as being able to hybridize under stringent conditions, to the nucleotide sequence shown in SEQ ID NO: 6, or a fragment of this sequence. Nucleic acid molecules corresponding to orthologs, homologs, and allelic variants of the 18480 cDNAs of the invention can further be isolated by mapping to the same chromosome or locus as the 18480 gene. Preferred variants include those that are correlated with protein kinase activity.

Allelic variants of 18480 e.g., human 18480, include both functional and non-functional proteins. Functional allelic variants are naturally occurring amino acid sequence variants of the 18480 protein within a population that maintain the ability, to modulate the phosphorylation state of itself or another protein or polypeptide. Functional allelic variants will typically contain only conservative substitution of one or more amino acids of SEQ ID NO:5, or substitution, deletion or insertion of non-critical residues in non-critical regions of the protein. Non-functional allelic variants are naturally-occurring amino acid sequence variants of the 18480 e.g., human 18480, protein within a population that do not have the ability to attach an acyl chain to a lipid precursor. Non-functional allelic variants will typically contain a non-conservative substitution, a deletion, or insertion, or premature truncation of the amino acid sequence of SEQ ID NO:5, or a substitution, insertion, or deletion in critical residues or critical regions of the protein.

Moreover, nucleic acid molecules encoding other 18480 family members and, thus, which have a nucleotide sequence which differs from the 18480 sequences of SEQ ID NO:4 or SEQ ID NO:6 are intended to be within the scope of the invention.

### Antisense Nucleic Acid Molecules, Ribozymes and Modified 18480 Nucleic Acid Molecules

In another aspect, the invention features, an isolated nucleic acid molecule which is antisense to 18480. An "antisense" nucleic acid can includes a nucleotide sequence which is complementary to a "sense" nucleic acid encoding a protein, e.g., complementary to the coding strand of a double-stranded cDNA molecule or complementary to an mRNA sequence. The antisense nucleic acid can be complementary to an entire 18480 coding strand, or to only a portion thereof (e.g., the coding region of human 18480 corresponding to SEQ ID NO:6). In another embodiment, the antisense nucleic acid molecule is antisense to a "noncoding region" of the coding strand of a nucleotide sequence encoding 18480 (e.g., the 5' and 3' untranslated regions).

An antisense nucleic acid can be designed such that it is complementary to the entire coding region of 18480 mRNA, but more preferably is an oligonucleotide which is antisense to only a portion of the coding or noncoding region of 18480 mRNA. For example, the antisense oligonucleotide can be complementary to the region surrounding the translation start site of 18480 mRNA, e.g., between the -10 and +10 regions of the target gene nucleotide sequence of interest. An antisense oligonucleotide can be, for example, about 7,10,15,20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding a 18480, protein, to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al., (1987) Nucleic Acids. Res. 15:6625-6641). The antisense nucleic acid molecule can also comprise a 2'-o-methylribonucleotide (Inoue et al., (1987) Nucleic Acids Res. 15:6131-6148) or a chimeric RNA-DNA analogue (Inoue et al., (1987) FEBS Lett. 215:327-330).

In still another embodiment, an antisense nucleic acid of the invention is a ribozyme. A ribozyme having specificity for a 18480-encoding nucleic acid can include one or more sequences complementary to the nucleotide sequence of a 18480 cDNA disclosed herein (i.e., SEQ ID NO:4 or SEQ ID NO:6), and a sequence having known catalytic sequence responsible for mRNA cleavage (see U.S. Pat. No. 5,093,246 or Haselhoff and Gerlach, (1988) Nature 334:585-591). For example, a derivative of a *Tetrahymena* L-19 IVS RNA can be constructed in which the nucleotide sequence of the active site is complementary to the nucleotide sequence to be cleaved in a 18480-encoding mRNA. See, e.g., Cech et al. U.S. Patent No. 4,987,071; and Cech et al. U.S. Patent No. 5,116,742. Alternatively, 18480 mRNA can be used to select a catalytic RNA having a specific ribonuclease activity from a pool of RNA molecules. See, e.g., Bartel, D. and Szostak, J.W. (1993) Science 261:1411-1418.

18480 gene expression can be inhibited by targeting nucleotide sequences complementary to the regulatory region of the 18480 (e.g., the 18480 promoter and/or enhancers) to form triple helical structures that prevent transcription of the 13237, 18480, 2245 or 16228 gene in target cells. See generally, Helene, C., (1991) Anticancer Drug Des. 6(6):569-84; Helene, C. et al., (1992) Ann. N. Y. Acad. Sci. 660:27-36; and Maher, L.J., (1992) Bioassays 14(12):807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

A 18480 nucleic acid molecule can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al., (1996) Bioorganic & Medicinal Chemistry 4 (1): 5-23). As used herein; the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. et al., (1996) *supra;* Perry-O'Keefe et al., Proc. Natl. Acad. Sci. 93: 14670-675.

PNAs of 18480 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 18480 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B., (1996) *supra*)); or as probes or primers for DNA sequencing or hybridization (Hyrup B. et al., (1996) *supra;* Perry-O'Keefe *supra*).

In other embodiments, the oligonucleotide may include other appended groups such as peptides (e.g., for targeting host cell receptors *in vivo*), or agents facilitating transport across the cell membrane (see, e.g., Letsinger et al., (1989) Proc. Natl. Acad. Sci. USA 86:6553-6556; Lemaitre et al., (1987) Proc. Natl. Acad. Sci. USA 84:648-652; PCT Publication No. W088/09810) or the blood-brain barrier (see, e.g., PCT Publication No. W089/10134). In addition, oligonucleotides can be modified with hybridization-triggered cleavage agents (See, e.g., Krol et al., (1988) Bio-Techniques 6:958-976) or intercalating agents. (See, e.g., Zon, (1988) Pharm. Res. 5:539-549). To this end, the oligonucleotide may be conjugated to another molecule, (e.g., a peptide, hybridization triggered crosslinking agent, transport agent, or hybridization-triggered cleavage agent).

The invention also includes molecular beacon oligonucleotide primer and probe molecules having at least one region which is complementary to a 18480 nucleic acid of the invention, two complementary regions one having a fluorophore and one a quencher such that the molecular beacon is useful for quantitating the presence of the 18480 nucleic acid of the invention in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

### Isolated 18480 Polypeptides

In another aspect, the invention features, an isolated 18480 protein, or fragment, e.g., a biologically active portion, for use as immunogens or antigens to raise or test (or more generally to bind) anti-, -18480 antibodies. 18480 protein can be isolated from cells or tissue sources using standard protein purification techniques. 18480 protein or fragments thereof can be produced by recombinant DNA techniques or synthesized chemically.

Polypeptides of the invention include those which arise as a result of the existence of multiple genes, alternative transcription events, alternative RNA splicing events, and alternative translational and postranslational events. The polypeptide can be expressed in systems, e.g., cultured cells, which result in substantially the same postranslational modifications present when expressed the polypeptide is expressed in a native cell, or in systems which result in the alteration or omission of postranslational modifications, e.g., gylcosylation or cleavage, present when expressed in a native cell.

In a preferred embodiment, a 18480 polypeptide has one or more of the following characteristics:
(i) it has the ability to reversibly phosphorylate proteins in order to regulate protein activity in eukaryotic cells;
(ii) it has a molecular weight, e.g., a deduced molecular weight, amino acid composition or other physical characteristic of the polypeptide of SEQ ID NO:5;
(iii) it has an overall sequence similarity of at least 80, 90, or 95%, with a polypeptide of SEQ ID NO:5;
(iv) it has an protein kinase domain which preferably has an overall sequence similarity of about 70%, 80%, 90% or 95% with SEQ ID NO:5;
(v) it has at least 70%, preferably 80%, and most preferably 95% of the cysteines found in the amino acid sequence of the native protein.

In a preferred embodiment the 18480 protein, or fragment thereof, differs from the corresponding sequence in SEQ ID NO:5. In one embodiment it differs by at least one but by less than 15, 10 or 5 amino acid residues. In another it differs from the corresponding sequence in SEQ ID NO:5, by at least one residue but less than 20%, 15%, 10% or 5% of the residues in it differ from the corresponding sequence in SEQ ID NO:5. (If this comparison requires alignment the sequences should be aligned for maximum homology. "Looped" out sequences from deletions or insertions, or mismatches, are considered differences.) The differences are, preferably, differences or changes at a non-essential residue or a conservative substitution. In a preferred embodiment the differences are not in the protein kinase domain. In another preferred embodiment one or more differences are in non-active site residues, e.g. outside of the protein kinase domain.

Other embodiments include a protein that contain one or more changes in amino acid sequence, e.g., a change in an amino acid residue which is not essential for activity. Such 18480 proteins differ in amino acid sequence from SEQ ID NO:5, yet retain biological activity.

In one embodiment, a biologically active portion of a 18480 protein includes an protein kinase domain. In another embodiment, a biologically active portion of a 18480 protein includes a casein kinase II phosphorylation domain. Moreover, other biologically active portions, in which other regions of the protein are deleted, can be prepared by recombinant techniques and evaluated for one or more of the functional activities of a native 18480 protein.

In a preferred embodiment, the 18480 protein has an amino acid sequence shown in SEQ ID NO:5. In other embodiments, the 18480 protein is substantially identical to SEQ ID NO:5. In yet another embodiment, the 18480 protein is substantially identical to SEQ ID NO:5 and retains the functional activity of the protein of SEQ ID NO:5, as described in detail above. Accordingly, in another embodiment, the 18480 protein is a protein which includes an amino acid sequence at least about 80%, 85%, 90%, 95%, 98% or more identical to SEQ ID NO:5.

### 18480 Chimeric or Fusion Proteins

In another aspect, the invention provides 18480 chimeric or fusion proteins. As used herein, a 18480 "chimeric protein" or "fusion protein" includes a 18480 polypeptide linked to a non- -18480 polypeptide. A "non -18480 polypeptide" refers to a polypeptide having an amino acid sequence corresponding to a protein which is not substantially homologous to the 18480 protein, e.g., a protein which is different from the 18480 protein and which is derived from the same or a different organism. The 18480 polypeptide of the fusion protein can correspond to all or a portion e.g., a fragment described herein of a 18480 amino acid sequence. In a preferred embodiment, a 18480 fusion protein includes at least one (or two) biologically active portion of a 18480 protein. The non-,-18480, polypeptide can be fused to the N-terminus or C-terminus of the 18480 polypeptide.

The fusion protein can include a moiety which has a high affinity for a ligand. For example, the fusion protein can be a GST- -18480, fusion protein in which the 18480, sequences are fused to the C-terminus of the GST sequences. Such fusion proteins can facilitate the purification of recombinant 18480. Alternatively, the fusion protein can be a 18480 protein containing a heterologous signal sequence at its N-terminus. In certain host cells (e.g., mammalian host cells), expression and/or secretion of 18480 can be increased through use of a heterologous signal sequence.

Fusion proteins can include all or a part of a serum protein, e.g., an IgG constant region, or human serum albumin.

The 18480 fusion proteins of the invention can be incorporated into pharmaceutical compositions and administered to a subject *in vivo.* The 18480 fusion proteins can be used to affect the bioavailability of a 18480 substrate. 18480 fusion proteins may be useful therapeutically for the treatment of disorders caused by, for example, (i) aberrant modification or mutation of a gene encoding a 18480 protein; (ii) mis-regulation of the 18480 gene; and (iii) aberrant post-translational modification of a 18480 protein.

Moreover, the 18480 fusion proteins of the invention can be used as immunogens to produce anti-, -18480 antibodies in a subject, to purify 18480 ligands and in screening assays to identify molecules which inhibit the interaction of 18480 with a 18480 substrate.

Expression vectors are commercially available that already encode a fusion moiety (e.g., a GST polypeptide). A 18480- -encoding nucleic acid can be cloned into such an expression vector such that the fusion moiety is linked in-frame to the 18480 protein.

### Variants of 18480 Proteins

In another aspect, the invention also features a variant of a 18480 polypeptide, e.g., which functions as an agonist (mimetics) or as an antagonist. Variants of the 18480 proteins can be generated by mutagenesis, e.g., discrete point mutation, the insertion or deletion of sequences or the truncation of a 18480 protein. An agonist of the 18480 proteins can retain substantially the same, or a subset, of the biological activities of the naturally occurring form of a 18480 protein. An antagonist of a 18480 protein can inhibit one or more of the activities of the naturally occurring form of the 18480 protein by, for example, competitively modulating a 18480- -mediated activity of a 18480 protein. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 18480 protein.

Variants of a 18480 protein can be identified by screening combinatorial libraries of mutants, e.g., truncation mutants, of a 18480 protein for agonist or antagonist activity.

Libraries of fragments e.g., N terminal, C terminal, or internal fragments, of a 18480 protein coding sequence can be used to generate a variegated population of fragments for screening and subsequent selection of variants of a 18480 protein.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property. Recursive ensemble mutagenesis (REM), a new technique which enhances the frequency of functional mutants in the libraries, can be used in combination with the screening assays to identify 13237, 18480, 2245 or 16228 variants (Arkin and Yourvan, (1992) Proc. Natal. Acad. Sci. USA 89:7811-7815; Delgrave et al., (1993) Protein Engineering 6(3):327-331).

Cell based assays can be exploited to analyze a variegated, 18480 library. For example, a library of expression vectors can be transfected into a cell line, e.g., a cell line, which ordinarily responds to 18480 in a substrate-dependent manner. The transfected cells are then contacted with 18480, and the effect of the expression of the mutant on signaling by the 18480 substrate can be detected, e.g., by measuring protein kinase activity. Plasmid DNA can then be recovered from the cells which score for inhibition, or alternatively, potentiation of signaling by the 18480 substrate, and the individual clones further characterized.

In another aspect, the invention features a method of making a 18480 polypeptide, e.g., a peptide having a non-wild type activity, e.g., an antagonist, agonist, or super agonist of a naturally occurring 18480 polypeptide, e.g., a naturally occurring 18480 polypeptide. The method includes: altering the sequence of a 18480 polypeptide, e.g., altering the sequence, e.g., by substitution or deletion of one or more residues of a non-conserved region, a domain or residue disclosed herein, and testing the altered polypeptide for the desired activity.

In another aspect, the invention features a method of making a fragment or analog of a 18480 polypeptide a biological activity of a naturally occurring 18480 polypeptide. The method includes: altering the sequence, e.g., by substitution or deletion of one or more residues, of a 18480 polypeptide, e.g., altering the sequence of a non-conserved region, or a domain or residue described herein, and testing the altered polypeptide for the desired activity.

### Anti- -18480, Antibodies

In another aspect, the invention provides an anti- 18480 antibody. The term "antibody" as used herein refers to an immunoglobulin molecule or immunologically active portion thereof, i.e., an antigen-binding portion. Examples of immunologically active portions of immunoglobulin molecules include F(ab) and F(ab')₂ fragments which can be generated by treating the antibody with an enzyme such as pepsin.

The antibody can be a polyclonal, monoclonal, recombinant, e.g., a chimeric or humanized, fully human, non-human, e.g., murine, or single chain antibody. In a preferred embodiment it has effector function and can fix complement. The antibody can be coupled to a toxin or imaging agent.

A full-length 18480 protein or, antigenic peptide fragment of 18480 can be used as an immunogen or can be used to identify anti--18480 antibodies made with other immunogens, e.g., cells, membrane preparations, and the like. The antigenic peptide of 18480 should include at least 8 amino acid residues of the amino acid sequence shown in SEQ ID NO:5 and encompasses an epitope of 18480. Preferably, the antigenic peptide includes at least 10 amino acid residues, more preferably at least 15 amino acid residues, even more preferably at least 20 amino acid residues, and most preferably at least 30 amino acid residues.

Fragments of 18480 which include, e.g., residues of SEQ ID NO:5 can be used to make, e.g., used as immunogens, or used to characterize the specificity of an antibody or antibodies against what are believed to be hydrophilic regions of the 18480 protein. Similarly, a fragment of 18480 can be used to make an antibody against what is believed to be a hydrophobic region of the 18480 protein; a fragment of 18480 can be used to make an antibody against the protein kinase region of the 18480 protein.

Antibodies reactive with, or specific for, any of these regions, or other regions or domains described herein are provided.

In a preferred embodiment the antibody fails to bind an Fc receptor, e.g. it is a type which does not support Fc receptor binding or has been modified, e.g., by deletion or other mutation, such that is does not have a functional Fc receptor binding region.

Preferred epitopes encompassed by the antigenic peptide are regions of 18480 are located on the surface of the protein, e.g., hydrophilic regions, as well as regions with high antigenicity. For example, an Emini surface probability analysis of the human 18480 protein sequence can be used to indicate the regions that have a particularly high probability of being localized to the surface of the 18480 protein and are thus likely to constitute surface residues useful for targeting antibody production.

In a preferred embodiment the antibody binds an epitope on any domain or region on 18480 proteins described herein.

Chimeric, humanized, but most preferably, completely human antibodies are desirable for applications which include repeated administration, e.g., therapeutic treatment (and some diagnostic applications) of human patients.

The anti- -18480, antibody can be a single chain antibody. A single-chain antibody (scFV) may be engineered (see, for example, Colcher, D. et al., Ann. NY Acad. Sci. 1999 Jun 30;880:263-80; and Reiter, Y., Clin. Cancer Res. 1996 Feb;2(2):245-52). The single chain antibody can be dimerized or multimerized to generate multivalent antibodies having specificities for different epitopes of the same target 18480 protein.

An anti- -18480 antibody (e.g., monoclonal antibody) can be used to isolate 18480 by standard techniques, such as affinity chromatography or immunoprecipitation. Moreover, an anti- 18480, antibody can be used to detect 18480 protein (e.g., in a cellular lysate or cell supernatant) in order to evaluate the abundance and pattern of expression of the protein. Anti- 1480 antibodies can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses

A vector can include a 18480 nucleic acid in a form suitable for expression of the nucleic acid in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 18480, proteins, mutant forms of 18480 proteins, fusion proteins, and the like).

The recombinant expression vectors of the invention can be designed for expression of 18480 proteins in prokaryotic or eukaryotic cells. For example, polypeptides of the invention can be expressed in E. coli, insect cells (e.g., using baculovirus expression vectors), yeast cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in E. coli with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S., (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be used in 18480 activity assays, (e.g., direct assays or competitive assays described in detail below), or to generate antibodies specific for 18480 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six (6) weeks).

To maximize recombinant protein expression in *E. coli* is to express the protein in host bacteria with an impaired capacity to proteolytically cleave the recombinant protein (Gottesman, S., Gene Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli* (Wada et al., (1992) Nucleic Acids Res. 20:2111-2118). Such alteration of nucleic acid sequences of the invention can be carried out by standard DNA synthesis techniques.

The 18480 expression vector can be a yeast expression vector, a vector for expression in insect cells, e.g., a baculovirus expression vector or a vector suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al., (1987) Genes Dev. 1:268-277), lymphoid-specific promoters (Calame and Eaton, (1988) Adv. Immunol. 43:235-275), in particular promoters of T cell receptors (Winoto and Baltimore, (1989) EMBO J. 8:729-733) and immunoglobulins (Banerji et al., (1983) Cell 33:729-740; Queen and Baltimore, (1983) Cell 33:741-748), neuron-specific promoters (e.g., the neurofilament promoter; Byrne and Ruddle, (1989) Proc. Natl. Acad. Sci. USA 86:5473-5477); pancreas-specific promoters (Edlund et al., (1985) Science 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss, (1990) Science 249:374-379) and the α-fetoprotein promoter (Campes and Tilghman, (1989) Genes Dev. 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid; phagemid or attenuated virus. For a discussion of the regulation of gene expression using antisense genes see Weintraub, H. et al., Antisense RNA as a molecular tool for genetic analysis, Reviews - Trends in Genetics, Vol. 1(1) 1986.

Another aspect the invention provides a host cell which includes a nucleic acid molecule described herein, e.g., a 18480 nucleic acid molecule within a recombinant expression vector or a 18480 nucleic acid molecule containing sequences which allow it to homologously recombine into a specific site of the host cell's genome. The terms "host cell" and "recombinant host cell" are used interchangeably herein. Such terms refer not only to the particular subject cell but rather also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a 18480, protein, can be expressed in bacterial cells such as *E. coli*, insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride coprecipitation, DEAE-dextran mediated transfection, lipofection, or electroporation

A host cell of the invention can be used to produce (i.e., express) a 18480, protein. Accordingly, the invention further provides methods for producing a 18480 protein using the host cells of the invention. In one embodiment, the method includes culturing the host cell of the invention (into which a recombinant expression vector encoding a 18480 protein has been introduced) in a suitable medium such that a 18480 protein is produced. In another embodiment, the method further includes isolating a 18480, protein from the medium or the host cell.

In another aspect, the invention features, a cell or purified preparation of cells which include a 18480 transgene, or which otherwise misexpress 18480. The cell preparation can consist of human or non-human cells, e.g., rodent cells, e.g., mouse or rat cells, rabbit cells, or pig cells. In preferred embodiments, the cell or cells include a 18480 transgene, e.g., a heterologous form of a 18480, e.g., a gene derived from humans (in the case of a non-human cell). The 18480 transgene can be misexpressed, e.g., overexpressed or underexpressed. In other preferred embodiment, the cell or cells include a gene which misexpress an endogenous 18480, e.g., a gene the expression of which is disrupted, e.g., a knockout. Such cells can serve as a model for studying disorders which are related to mutated or mis-expressed 18480 alleles or for use in drug screening.

In another aspect, the invention features, a human cell, e.g., a hematopoietic stem cell, transformed with nucleic acid which encodes a subject 18480 polypeptide.

Also provided are cells or a purified preparation thereof, e.g., human cells, in which an endogenous 18480 is under the control of a regulatory sequence that does not normally control the expression of the endogenous 18480 gene. The expression characteristics of an endogenous gene within a cell, e.g., a cell line or microorganism, can be modified by inserting a heterologous DNA regulatory element into the genome of the cell such that the inserted regulatory element is operably linked to the endogenous 18480 gene. For example, an endogenous

18480 gene, e.g., a gene which is "transcriptionally silent," e.g., note normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published on May 16, 1991.

### Uses

The nucleic acid molecules, proteins, protein homologues, and antibodies described herein can be used in one or more of the following methods: a) screening assays; and b) predictive medicine (e.g., diagnostic assays, prognostic assays, monitoring clinical trials, and pharmacogenetics).

The isolated nucleic acid molecules of the invention can be used, for example, to express a 18480 protein (e.g., via a recombinant expression vector in a host cell in gene therapy applications), to detect a 18480 mRNA (e.g., in a biological sample) or a genetic alteration in a 18480, gene, and to modulate 18480 activity, as described further below. The 18480, proteins can be used to treat disorders characterized by insufficient or excessive production of a 18480 substrate or production of 18480 inhibitors. In addition, the 18480 proteins can be used to screen for naturally occurring 18480 substrates, to screen for drugs or compounds which modulate 18480 activity, as well as to treat disorders characterized by insufficient or excessive production of 18480 protein or production of 18480 protein forms which have decreased, aberrant or unwanted activity compared to 18480 wild-type protein. Such disorders include those characterized by aberrant signaling or aberrant, e.g., hyperproliferative, cell growth. Moreover, the anti -18480, antibodies of the invention can be used to detect and isolate 18480 proteins, regulate the bioavailability of 18480 proteins, and modulate 18480 activity.

A method of evaluating a compound for the ability to interact with, e.g., bind, a subject 18480 polypeptide is provided. The method includes: contacting the compound with the subject 18480 polypeptide; and evaluating ability of the compound to interact with, e.g., to bind or form a complex with the subject 18480 polypeptide. This method can be performed in vitro, e.g., in a cell free system, or *in vivo,* e.g., in a two-hybrid interaction trap assay. This method can be used to identify naturally occurring molecules which interact with subject, 18480 polypeptide. It can also be used to find natural or synthetic inhibitors of subject, 18480 polypeptide. Screening methods are discussed in more detail below.

### Screening Assays:

The invention provides methods (also referred to herein as "screening assays") for identifying modulators, i.e., candidate or test compounds or agents (e.g., proteins, peptides, peptidomimetics, peptoids, small molecules or other drugs) which bind to 18480, proteins, have a stimulatory or inhibitory effect on, for example, 18480 expression or 18480 activity, or have a stimulatory or inhibitory effect on, for example, the expression or activity of a 18480 substrate. Compounds thus identified can be used to modulate the activity of target gene products (e.g., 18480 genes) in a therapeutic protocol, to elaborate the biological function of the target gene product, or to identify compounds that disrupt normal target gene interactions.

In one embodiment, the invention provides assays for screening candidate or test compounds which are substrates of a 18480, protein or polypeptide or a biologically active portion thereof. In another embodiment, the invention provides assays for screening candidate or test compounds which bind to or modulate the activity of a 18480 protein or polypeptide or a biologically active portion thereof.

The test compounds of the present invention can be obtained using any of the numerous approaches in combinatorial library methods known in the art, including: biological libraries; peptoid libraries [libraries of molecules having the functionalities of peptides, but with a novel, non-peptide backbone which are resistant to enzymatic degradation but which nevertheless remain bioactive] (see, e.g., Zuckermann, R.N. et al., J. Med. Chem. 1994, 37: 2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al., (1994) Proc. Natl. Acad. Sci. USA 91:11422; Zuckermann et al., (1994). J. Med. Chem. 37:2678; Cho et al., (1993) Science 261:1303; Carrell et al., (1994) Angew. Chem. Int. Ed. Engl. 33:2059; Carell et al., (1994) Angew. Chem. Int. Ed. Engl. 33:2061; and in Gallop et al., (1994) J. Med. Chem. 37:1233.

Libraries of compounds may be presented in solution (e.g., Houghten, (1992) Biotechniques 13:412-421), or on beads (Lam, (1991) Nature 354: 82-84), chips (Fodor, (1993) Nature 364:555-556), bacteria or spores (Ladner, United States Patent No. 5,223,409), plasmids (Cull et al., (1992) Proc. Natal. Acad. Sci. USA 89:1865-1869) or on phage (Scott and Smith, (1990) Science 249:386-390); (Devlin, (1990) Science 249:404-406); (Cwirla et al., (1990) Proc. Natl. Acad. Sci. 87:6378-6382); (Felici, (1991) J. Mol. Biol. 222:301-310); (Ladner *supra*.). In one embodiment, an assay is a cell-based assay in which a cell which expresses a 18480 protein or biologically active portion thereof is contacted with a test compound, and the ability of the test compound to modulate 18480 activity is determined. Determining the ability of the test compound to modulate 18480 activity can be accomplished by monitoring, for example, protein kinase activity. The cell, for example, can be of mammalian origin, e.g., human. Cell homogenates, or fractions, preferably membrane containing fractions, can also be tested.

The ability of the test compound to modulate 18480 binding to a compound, e.g., a 18480 substrate, or to bind to 18480, can also be evaluated. This can be accomplished, for example, by coupling the compound, e.g., the substrate, with a radioisotope or enzymatic label such that binding of the compound, e.g., the substrate, to 18480, can be determined by detecting the labeled compound, e.g., substrate, in a complex. Alternatively, 18480, could be coupled with a radioisotope or enzymatic label to monitor the ability of a test compound to modulate 18480, binding to a 18480 substrate in a complex. For example, compounds (e.g., 18480 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 18480 substrate) to interact with 18480 with or without the labeling of any of the interactants can be evaluated. For example, a microphysiometer can be used to detect the interaction of a compound with 18480, without the labeling of either the compound or the 18480. McConnell, H. M. et al., (1992) Science 257:1906-1912. As used herein, a "microphysiometer". (e.g., Cytosensor) is an analytical instrument that measures the rate at which a cell acidifies its environment using a light-addressable potentiometric sensor (LAPS). Changes in this acidification rate can be used as an indicator of the interaction between a compound and 18480.

In yet another embodiment, a cell-free assay is provided in which a 18480, protein or biologically active portion thereof is contacted with a test compound and the ability of the test compound to bind to the 18480, protein or biologically active portion thereof is evaluated. Preferred biologically active portions of the 18480 proteins to be used in assays of the present invention include fragments which participate in interactions with non- -18480, -molecules, e.g., fragments with high surface probability scores.

Soluble and/or membrane-bound forms of isolated proteins (e.g., 18480, proteins or biologically active portions thereof) can be used in the cell-free assays of the invention. When membrane-bound forms of the protein are used, it may be desirable to utilize a solubilizing agent. Examples of such solubilizing agents include nonionic detergents such as n-octylglucoside, n-dodecylglucoside, n-dodecylmaltoside, octanoyl-N-methylglucamide, decanoyl-N-methylglucamide, Triton^{®} X-100, Triton^{®} X-114, Thesit^{®}, Isotridecypoly(ethylene glycol ether)ₙ, 3-[(3-cholamidopropyl)dimethylamminio]-1-propane sulfonate (CHAPS), 3-[(3-cholamidopropyl)dimethylammino]-2-hydroxy-1-propane sulfonate (CHAPSO), or N-dodecyl-N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

In one embodiment, assays are performed where the ability of an agent to block protein kinase activity within a cell is evaluated.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 18480, protein to bind to a target molecule can be accomplished using real-time Biomolecular Interaction Analysis (BIA) (see, e.g., Sjolander, S. and Urbaniczky, C., (1991) Anal. Chem. 63:2338-2345 and Szabo et al., (1995) Curr. Opin. Struct. Biol. 5:699-705). "Surface plasmon resonance" or "BIA" detects biospecific interactions in real time, without labeling any of the interactants (e.g.; BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize either 18480, an anti--18480 antibody or its target molecule to facilitate separation of complexed from uncomplexed forms of one or both of the proteins, as well as to accommodate automation of the assay. Binding of a test compound to a 18480, protein, or interaction of a 18480 protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase 18480, fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 18480 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 18480 binding or activity determined using standard techniques.

Other techniques for immobilizing either a 18480 protein or a target molecule on matrices include using conjugation of biotin and streptavidin. Biotinylated 18480, protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 18480 protein or target molecules but which do not interfere with binding of the 18480 protein to its target molecule. Such antibodies can be derivatized to the wells of the plate, and unbound target or 18480 protein trapped in the wells by antibody conjugation. Methods for detecting such complexes, in addition to those described above for the GST-immobilized complexes, include immunodetection of complexes using antibodies reactive with the 18480, protein or target molecule, as well as enzyme-linked assays which rely on detecting an enzymatic activity associated with the 18480 protein or target molecule.

Alternatively, cell free assays can be conducted in a liquid phase. In such an assay, the reaction products are separated from unreacted components, by any of a number of standard techniques, including but not limited to: differential centrifugation (see, for example, Rivas, G., and Minton, A.P., Trends Biochem Sci 1993 Aug;18(8):284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. Current Protocols in Molecular Biology 1999, J. Wiley: New York). Such resins and chromatographic techniques are known to one skilled in the art (see, e.g., Heegaard, N.H., J Mol. Recognit. 1998 Winter;11(1-6):141-8; Hage, D.S., and Tweed, S.A., J. Chromatogr. B Biomed. Sci. Appl. 1997 Oct 10;699(1-2):499-525). Further, fluorescence energy transfer may also be conveniently utilized, as described herein, to detect binding without further purification of the complex from solution.

In a preferred embodiment, the assay includes contacting the 18480, protein or biologically active portion thereof with a known compound which binds 18480, to form an assay mixture, contacting the assay mixture with a test compound, and determining the ability of the test compound to interact with a 13237, 18480 protein, wherein determining the ability of the test compound to interact with a 18480 protein includes determining the ability of the test compound to preferentially bind to 18480, or biologically active portion thereof, or to modulate the activity of a target molecule, as compared to the known compound.

The target gene products of the invention can, *in vivo,* interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to molecules such as antibodies, peptides, and small molecules. The preferred target genes/products for use in this embodiment are the 18480 genes herein identified. In an alternative embodiment, the invention provides methods for determining the ability of the test compound to modulate the activity of a 18480, protein through modulation of the activity of a downstream effector of a 18480 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), e.g., a substrate, a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described below.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by non-covalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 18480 proteins can be used as "bait proteins" in a two-hybrid assay or three-hybrid assay (see, e.g., U.S. Patent No. 5,283,317; Zérvos et al., (1993) Cell 72:223-232; Madura et al., (1993) J. Biol. Chem. 268:12046-12054; Bartel et al., (1993) Biotechniques 14:920-924; Iwabuchi et al., (1993) Oncogene 8:1693-1696; and Brent WO94/10300), to identify other proteins, which bind to or interact with 18480" -, 18480-, binding proteins" or "18480- -bp") and are involved in 18480 activity. Such 18480- bps can be activators or inhibitors of signals by the 18480 proteins or 18480 targets as, for example, downstream elements of a 18480- mediated signaling pathway.

The two-hybrid system is based on the modular nature of most transcription factors, which consist of separable DNA-binding and activation domains. Briefly, the assay utilizes two different DNA constructs. In one construct, the gene that codes for a 18480, protein is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 18480 protein can be the fused to the activator domain.) If the "bait" and the "prey" proteins are able to interact, *in vivo*, forming a 18480-, or dependent complex, the DNA-binding and activation domains of the transcription factor are brought into close proximity. This proximity allows transcription of a reporter gene (e.g., LacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 18480 protein.

In another embodiment, modulators of 18480 expression are identified. For example, a cell or cell free mixture is contacted with a candidate compound and the expression of 18480 mRNA or protein evaluated relative to the level of expression of 18480 mRNA or protein in the absence of the candidate compound. When expression of 18480 mRNA or protein is greater in the presence of the candidate compound than in its absence, the candidate compound is identified as a stimulator of, 18480 mRNA or protein expression. Alternatively, when expression of 18480, mRNA or protein is less (statistically significantly less) in the presence of the candidate compound than in its absence, the candidate compound is identified as an inhibitor of 18480, mRNA or protein expression. The level of 18480, mRNA or protein expression can be determined by methods described herein for detecting, 18480, mRNA or protein.

In another aspect, the invention pertains to a combination of two or more of the assays described herein. For example, a modulating agent can be identified using a cell-based or a cell free assay, and the ability of the agent to modulate the activity of a 18480 protein can be confirmed *in vivo,* e.g., in an animal.

This invention further pertains to novel agents identified by the above-described screening assays. Accordingly, it is within the scope of this invention to further use an agent identified as described herein (e.g., a 18480 modulating agent, an antisense 18480 nucleic acid molecule, a 18480 -specific antibody, or a 18480 -binding partner) in an appropriate animal model to determine the efficacy, toxicity, side effects, or mechanism of action, of treatment with such an agent. Furthermore, novel agents identified by the above-described screening assays can be used for treatments as described herein.

### Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 18480.

Such disorders include, e.g., a disorder associated with the misexpression of 18480 or lipid metabolism related disorder.

The method includes one or more of the following:
detecting, in a tissue of the subject, the presence or absence of a mutation which affects the expression of the 18480 gene, or detecting the presence or absence of a mutation in a region which controls the expression of the gene, e.g., a mutation in the 5' control region;
detecting, in a tissue of the subject, the presence or absence of a mutation which alters the structure of the 18480 gene;
detecting, in a tissue of the subject, the misexpression of the 18480 gene, at the mRNA level, e.g., detecting a non-wild type level of a mRNA ;
detecting, in a tissue of the subject, the misexpression of the gene, at the protein level, e.g., detecting a non-wild type level of a 18480 polypeptide.

In preferred embodiments the method includes: ascertaining the existence of at least one of: a deletion of one or more nucleotides from the 18480 gene; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:4 naturally occurring mutants thereof or 5' or 3' flanking sequences naturally associated with the 18480 gene; (ii) exposing the probe/primer to nucleic acid of the tissue; and detecting, by hybridization, *e.g*., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 18480 gene; the presence of a non-wild type splicing pattern of a messenger RNA transcript of the gene; or a non-wild type level of 18480.

Methods of the invention can be used prenatally or to determine if a subject's offspring will be at risk for a disorder.

In preferred embodiments the method includes determining the structure of a 18480 gene, an abnormal structure being indicative of risk for the disorder.

In preferred embodiments the method includes contacting a sample form the subject with an antibody to the 18480 protein or a nucleic acid, which hybridizes specifically with the gene. These and other embodiments are discussed below.

### Diagnostic and Prognostic Assays

The presence, level, or absence of 18480 protein or nucleic acid in a biological sample can be evaluated by obtaining a biological sample from a test subject and contacting the biological sample with a compound or an agent capable of detecting 18480 protein or nucleic acid (e.g., mRNA genomic DNA) that encodes 18480 protein such that the presence of 18480 protein or nucleic acid is detected in the biological sample. The term "biological sample" includes tissues, cells and biological fluids isolated from a subject, as well as tissues, cells and fluids present within a subject. A preferred biological sample is serum. The level of expression of the 18480 gene can be measured in a number of ways, including, but not limited to: measuring the mRNA encoded by the 18480 genes; measuring the amount of protein encoded by the 18480 genes; or measuring the activity of the protein encoded by the 18480 genes.

The level of mRNA corresponding to the 18480 gene in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, a full-length 18480 nucleic acid, such as the nucleic acid of SEQ ID NO:4, or a portion thereof, such as an oligonucleotide of at least 7, 15, 30, 50, 100, 250 or 500 nucleotides in length and sufficient to specifically hybridize under stringent conditions to 18480, mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the RNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level ofmRNA encoded by the 18480 genes.

The level of mRNA in a sample that is encoded by one of 18480 can be evaluated with nucleic acid amplification, e.g., byrtPCR (Mullis, 1987, U.S. Patent No. 4,683,202), ligase chain reaction (Barany, 1991, Proc. Natl. Acad. Sci. USA 88:189-193), self sustained sequence replication (Guatelli et al., 1990, Proc. Natl. Acad. Sci. USA 87:1874-1878), transcriptional amplification system (Kwoh et al., 1989, Proc. Natal. Acad Sci. USA 86: 1173-1177), Q-Beta Replicase (Lizardi et al., 1988, Bio/Technology 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 18480 gene being analyzed.

In another embodiment, the methods further contacting a control sample with a compound or agent capable of detecting 18480 mRNA, or genomic DNA, and comparing the presence of 18480 mRNA or genomic DNA in the control sample with the presence of 18480 mRNA or genomic DNA in the test sample. A variety of methods can be used to determine the level of protein encoded by 18480. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 18480 protein in a biological sample *in vitro* as well as *in vivo. In vitro* techniques for detection of 18480 protein include enzyme linked immunosorbent assays (ELISAs), immunoprecipitations, immunofluorescence, enzyme immunoassay (EIA), radioimmunoassay (RIA), and Western blot analysis. *In vivo* techniques for detection of 18480, protein include introducing into a subject a labeled anti--18480 antibody. For example, the antibody can be labeled with a radioactive marker whose presence and location in a subject can be detected by standard imaging techniques.

In another embodiment, the methods further include contacting the control sample with a compound or agent capable of detecting 18480 protein, and comparing the presence of 18480 protein in the control sample with the presence of 18480 protein in the test sample.

The invention also includes kits for detecting the presence of 18480 in a biological sample. For example, the kit can include a compound or agent capable of detecting 18480 protein or mRNA in a biological sample; and a standard. The compound or agent can be packaged in a suitable container. The kit can further comprise instructions for using the kit to detect 18480 protein or nucleic acid.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention or (2) a pair of primers useful for amplifying a nucleic acid molecule corresponding to a marker of the invention. The kit can also includes a buffering agent, a preservative, or a protein-stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 18480 expression or activity. As used herein, the term "unwanted" includes an unwanted phenomenon involved in a biological response such as pain or deregulated cell proliferation. In one embodiment, a disease or disorder associated with aberrant or unwanted 18480 expression or activity is identified. A test sample is obtained from a subject and 18480 protein or nucleic acid (e.g., mRNA or genomic DNA) is evaluated, wherein the level, e.g., the presence or absence, of 18480 protein or nucleic acid is diagnostic for a subject having or at risk of developing a disease or disorder associated with aberrant or unwanted 18480 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 18480 expression or activity. For example, such methods can be used to determine whether a subject can be effectively treated with an agent for a cellular growth related disorder. The methods of the invention can also be used to detect genetic alterations in a 18480 gene, thereby determining if a subject with the altered gene is at risk for a disorder characterized by misregulation in 18480 protein activity or nucleic acid expression, such as a cellular growth related disorder. In preferred embodiments, the methods include detecting, in a sample from the subject, the presence or absence of a genetic alteration characterized by at least one of an alteration affecting the integrity of a gene encoding a 18480 -protein, or the mis-expression of the 18480 gene. For example, such genetic alterations can be detected by ascertaining the existence of at least one of 1) a deletion of one or more nucleotides from a 18480 gene; 2) an addition of one or more nucleotides to a 18480 gene; 3) a substitution of one or more nucleotides of a 18480 gene, 4) a chromosomal rearrangement of a 18480 gene; 5) an alteration in the level of a messenger RNA transcript of a 18480 gene, 6) aberrant modification of a 18480 gene, such as of the methylation pattern of the genomic DNA, 7) the presence of a non-wild type splicing pattern of a messenger RNA transcript of a 18480 gene, 8) a non-wild type level of a 18480- protein, 9) allelic loss of a 18480 gene, and 10) inappropriate post-translational modification of a 18480 protein.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 18480- gene. This method can include the steps of collecting a sample of cells from a subject, isolating nucleic acid (e.g., genomic, mRNA or both) from the sample, contacting the nucleic acid sample with one or more primers which specifically hybridize to a 18480 gene under conditions such that hybridization and amplification of the 18480 -gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., (1990) Proc. Natl. Acad. Sci. URSA 87:1874-1878), transcriptional amplification system (Kwoh, D.Y. et al., (1989) Proc. Natl. Acad. Sci. USA 86:1173-1177), Q-Beta Replicase (Lizardi, P.M. et al., (1988) Bio-Technology 6:1197), or other nucleic acid amplification methods, followed by the detection of the amplified molecules using techniques known to those of skill in the art.

In another embodiment, mutations in a 18480 gene from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 18480 can be identified by hybridizing a sample and control nucleic acids, e.g., DNA or RNA, two-dimensional arrays, e.g., chip based arrays. Such arrays include a plurality of addresses, each of which is positionally distinguishable from the other. A different probe is located at each address of the plurality. The arrays can have a high density of addresses, e.g., can contain hundreds or thousands of oligonucleotides probes (Cronin, M.T. et al., (1996) Human Mutation 7: 244-255; Kozal, M.J. et al., (1996) Nature Medicine 2:753-759). For example, genetic mutations in 18480 can be identified in two dimensional arrays containing light-generated DNA probes as described in Cronin, M.T. et al., *supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 18480 gene and detect mutations by comparing the sequence of the sample 18480, with the corresponding wild-type (control) sequence. Automated sequencing procedures can be utilized when performing the diagnostic assays ((1995) Biotechniques 19:448), including sequencing by mass spectrometry.

Other methods for detecting mutations in the 18480 gene include methods in which protection from cleavage agents is used to detect mismatched bases in RNA/RNA or RNA/DNA heteroduplexes (Myers et al., (1985) Science 230:1242; Cotton et al., (1988) Proc. Natl. Acad. Sci. USA 85:4397; Saleeba et al., (1992) Methods Enzymol. 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 18480 cDNAs obtained from samples of cells. For example, the mutY enzyme of *E. coli* cleaves A at G/A mismatches and the thymidine DNA glycosylase from HeLa cells cleaves T at G/T mismatches (Hsu et al., (1994) Carcinogenesis 15:1657-1662; U.S. Patent No. 5,459,039).

In other embodiments, alterations in electrophoretic mobility will be used to identify mutations in 18480 genes. For example, single strand conformation polymorphism (SSCP) may be used to detect differences in electrophoretic mobility between mutant and wild type nucleic acids (Orita et al., (1989) Proc. Natl. Acad. Sci. USA: 86:2766, see also Cotton, (1993) Mutat. Res. 285:125-144; and Hayashi, (1992) Genet. Anal. Tech. Appl. 9:73-79). Single-stranded DNA fragments of sample and control 18480 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al., (1991) Trends Genet. 7:5).

In yet another embodiment, the movement of mutant or wild-type fragments in polyacrylamide gels containing a gradient of denaturant is assayed using denaturing gradient gel electrophoresis (DGGE) (Myers et al., (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner, (1987) Biophys. Chem. 265:12753).

Examples of other techniques for detecting point mutations include, but are not limited to, selective oligonucleotide hybridization, selective amplification, or selective primer extension (Saiki et al., (1986) Nature 324:163); Saiki et al., (1989) Proc. Natl. Acad. Sci. USA 86:6230).

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et aL, (1989) Nucleic Acids Res. 17:2437-2448) or at the extreme 3' end of one primer where, under appropriate conditions, mismatch can prevent, or reduce polymerase extension (Prossner, (1993) Tibtech 11:238). In addition it may be desirable to introduce a novel restriction site in the region of the mutation to create cleavage-based detection (Gasparini et al., (1992) Mol. Cell Probes 6:1). It is anticipated that in certain embodiments amplification may also be performed using Taq ligase for amplification (Barany, (1991) Proc. Natl. Acad. Sci USA 88:189). In such cases, ligation will occur only if there is a perfect match at the 3' end of the 5' sequence making it possible to detect the presence of known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g*., in clinical settings to diagnose patients exhibiting symptoms or family history of a disease or illness involving a 18480 gene.

This invention is further illustrated by the following examples which should not be construed as limiting. The contents of all references, patents and published patent applications cited throughout this application are incorporated herein by reference.

### EXAMPLES

### Example 1: Identification and Characterization of Human 18480 cDNAs

The human 18480 sequence (Figure 6A-C, SEQ ID NO:4), which is approximately 2438 nucleotides long including untranslated regions, contains a predicted methionine-initiated coding sequence of about 2079 nucleotides (nucleotides 45-2123 of SEQ ID NO:4, SEQ ID NO:6). The coding sequence encodes a 692 amino acid protein (SEQ ID NO:5).

### Example 2: Tissue Distribution of 18480 mRNA

Northern blot hybridizations with various RNA samples can be performed under standard conditions and washed under stringent conditions, i.e., 0.2xSSC at 65°C. A DNA probe corresponding to all or a portion of the 18480 cDNA (SEQ ID NO:4) or 18480 cDNA can be used. The DNA was radioactively labeled with ³²P-dCTP using the Prime-It Kit (Stratagene, La Jolla, CA) according to the instructions of the supplier. Filters containing mRNA from mouse hematopoietic and endocrine tissues, and cancer cell lines (Clontech, Palo Alto, CA) can be probed in ExpressHyb hybridization solution (Clontech) and washed at high stringency according to manufacturer's recommendations.

### Example 3: Gene Expression Analysis

Total RNA was prepared from various human tissues by a single step extraction method using RNA STAT-60 according to the manufacturer's instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using β-2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human 18480 expression was measured by TaqMan® quantitative PCR (Perkin Elmer Applied Biosystems) in cDNA prepared from a variety of normal and diseased (e.g., cancerous) human tissues or cell lines.

Probes were designed by PrimerExpress software (PE Biosystems) based on the sequence of the human 18480 gene. Each human 18480, gene probe was labeled using FAM (6-carboxyfluorescein), and the β2-microglobulin reference probe was labeled with a different fluorescent dye, VIC. The differential labeling of the target gene and internal reference gene thus enabled measurement in same well. Forward and reverse primers and the probes for both β2-microglobulin and target gene were added to the TaqMan® Universal PCR Master Mix (PE Applied Biosystems). Although the final concentration of primer and probe could vary, each was internally consistent within a given experiment. A typical experiment contained 200nM of forward and reverse primers plus 100nM probe for β-2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 18480, gene expression in the various tissues relative to β-2 microglobulin expression in the same tissue. The threshold cycle (Ct) value is defined as the cycle at which a statistically significant increase in fluorescence is detected. A lower Ct value is indicative of a higher mRNA concentration. The Ct value of the human 18480, gene is normalized by subtracting the Ct value of the β-2 microglobulin gene to obtain a ACt value using the following formula: ΔCt=Ctₕᵤₘₐₙ 59914 and 59921 - Ct β-2 microglobulin. Expression is then calibrated against a cDNA sample showing a comparatively low level of expression of the human 18480 gene. The ΔCt value for the calibrator sample is then subtracted from ΔCt for each tissue sample according to the following formula: ΔΔCt=ΔCt-ₛₐₘₚₗₑ - ΔCt-_{calibrator}. Relative expression is then calculated using the arithmetic formula given by 2-ΔΔCt. Expression of the target human 18480 gene in each of the tissues tested is then graphically represented as discussed in more detail below.

TaqMan real-time quantitative RT-PCR is used to detect the presence of RNA transcript corresponding to human 18480 relative to a no template control in a panel of human tissues or cells. It is found that the highest expression of 18480 orthologs are expressed in HUVEC as shown in the following Table 2.

**Table 2**

| Phase 1.5.2 Expression of 18480 | | | | |
|---|---|---|---|---|
| Tissue Type | Mean | β 2 Mean | ∂∂ Ct | Expression |
| Artery normal | 34.26 | 23.27 | 10.99 | 0.4917 |
| Aorta diseased | 32.16 | 22.72 | 9.44 | 1.4397 |
| Vein normal | 35.98 | 20.28 | 15.7 | 0 |
| Coronary SMC | 33.06 | 23.07 | 9.98 | 0.9868 |
| HUVEC | 27.28 | 22.07 | 5.21 | 26.9233 |
| Hemangioma | 29.09 | 20.11 | 8.97 | 1.9873 |
| Heart normal | 31.45 | 20.93 | 10.53 | 0.6787 |
| Heart CHF | 31.4 | 20.02 | 11.39 | 0.3739 |
| Kidney | 26.52 | 20.7 | 5.82 | 17.7628 |
| Skeletal Muscle | 33.1 | 23.08 | 10.03 | 0.9598 |
| Adipose normal | 35.04 | 21.09 | 13.95 | 0 |
| Pancreas | 29.88 | 21.98 | 7.91 | 4.1721 |
| primary osteoblasts | 33.88 | 21.05 | 12.84 | 0.1369 |
| Osteoclasts (diff) | 34.31 | 17.9 | 16.41 | 0.0114 |
| Skin normal | 31.06 | 21.95 | 9.1 | 1.8223 |
| Spinal cord normal | 34.17 | 21.14 | 13.03 | 0.1196 |
| Brain Cortex normal | 30.76 | 22.25 | 8.51 | 2.7431 |
| Brain Hypothalamus normal | 30.31 | 22.2 | 8.11 | 3.6321 |
| Nerve | 35.23 | 22.4 | 12.84 | 0 |
| DRG (Dorsal Root Ganglion) | 31.22 | 22.25 | 8.96 | 2.0011 |
| Breast normal | 30.93 | 21.04 | 9.88 | 1.0576 |
| Breast tumor | 31.63 | 21.29 | 10.34 | 0.7715 |
| Ovary normal | 28.89 | 20.61 | 8.27 | 3.2395 |
| Ovary Tumor | 29.23 | 20.68 | 8.55 | 2.6588 |
| Prostate Normal | 28.4 | 20.13 | 8.27 | 3.2395 |
| Prostate Tumor | 28.95 | 20.78 | 8.16 | 3.4841 |
| Salivary glands | 32.22 | 19.95 | 12.27 | 0.2032 |
| Colon normal | 30.91 | 18.66 | 12.25 | 0.2053 |
| Colon Tumor | 27.34 | 19.31 | 8.03 | 3.8391 |
| Lung normal | 29.87 | 18.49 | 11.38 | 0.3752 |
| Lung tumor | 26.93 | 20.66 | 6.27 | 12.9581 |
| Lung COPD | 28.62 | 18.75 | 9.87 | 1.0686 |
| Colon IBD | 30.18 | 17.99 | 12.2 | 0.2133 |
| Liver normal | 30.22 | 20.32 | 9.9 | 1.043 |
| Liver fibrosis | 30.48 | 22.11 | 8.38 | 3.0121 |
| Spleen normal | 28.82 | 20.19 | 8.63 | 2.5329 |
| Tonsil normal | 26.12 | 17.67 | 8.45 | 2.8595 |
| Lymph node normal | 28.45 | 19.48 | 8.97 | 1.9873 |
| Small intestine normal | 34.67 | 20.89 | 13.78 | 0.0711 |
| Skin-Decubitus | 34.37 | 21.63 | 12.74 | 0.1457 |
| Synovium | 30.85 | 20.01 | 10.84 | 0.5456 |
| BM-MNC | 35.14 | 19.09 | 16.05 | 0 |
| Activated PBMC | 32.8 | 18.17 | 14.63 | 0.0396 |
| Neutrophils | 32.72 | 19.48 | 13.23 | 0.1037 |
| Megakaryocytes | 30.06 | 19.19 | 10.87 | 0.5343 |
| Erythroid | 29.23 | 21.63 | 7.6 | 5.1543 |

### Example 5: Recombinant Expression of 18480 in Bacterial Cells

In this example, 18480 is expressed as a recombinant glutathione-S-transferase (GST) fusion polypeptide in *E. coli* and the fusion polypeptide is isolated and characterized. Specifically, 18480 is fused to GST and this fusion polypeptide is expressed in *E. coli,* e.g., strain PEB 199. Expression of the GST--18480 fusion protein in PEB199 is induced with IPTG. The recombinant fusion polypeptide is purified from crude bacterial lysates of the induced PEB199 strain by affinity chromatography on glutathione beads. Using polyacrylamide gel electrophoretic analysis of the polypeptide purified from the bacterial lysates, the molecular weight of the resultant fusion polypeptide is determined.

### Example 6: Expression of Recombinant, 18480 Protein in COS Cells

To express the 18480 gene in COS cells, the pcDNA/Amp vector by Invitrogen Corporation (San Diego, CA) is used. This vector contains an SV40 origin of replication, an ampicillin resistance gene, an *E. coli* replication origin, a CMV promoter followed by a polylinker region, and an SV40 intron and polyadenylation site. A DNA fragment encoding the entire 18480 protein and an HA tag (Wilson et al. (1984) Cell 37:767) or a FLAG tag fused in-frame to its 3' end of the fragment is cloned into the polylinker region of the vector, thereby placing the expression of the recombinant protein under the control of the CMV promoter.

To construct the plasmid, the 18480 DNA sequence is amplified by PCR using two primers. The 5' primer contains the restriction site of interest followed by approximately twenty nucleotides of the 18480 coding sequence starting from the initiation codon; the 3' end sequence contains complementary sequences to the other restriction site of interest, a translation stop codon, the HA tag or FLAG tag and the last 20 nucleotides of the 18480 coding sequence. The PCR amplified fragment and the pCDNA/Amp vector are digested with the appropriate restriction enzymes and the vector is dephosphorylated using the CIAP enzyme (New England Biolabs, Beverly, MA). Preferably the two restriction sites chosen are different so that the 18480 gene is inserted in the correct orientation. The ligation mixture is transformed into *E. coli* cells (strains HB101, DH5α, SURE, available from Stratagene Cloning Systems, La Jolla, CA, can be used), the transformed culture is plated on ampicillin media plates, and resistant colonies are selected. Plasmid DNA is isolated from transformants and examined by restriction analysis for the presence of the correct fragment.

COS cells are subsequently transfected with the 18480- pcDNA/Amp plasmid DNA using the calcium phosphate or calcium chloride coprecipitation methods, DEAE-dextran-mediated transfection, lipofection, or electroporation. Other suitable methods for transfecting host cells can be found in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989. The expression of the 18480 polypeptide is detected by radiolabelling (³⁵S-methionine or ³⁵S-cysteine available from NEN, Boston, MA, can be used) and immunoprecipitation (Harlow, E. and Lane, D. Antibodies: A Laboratory Manual, Cold Spring Harbor Laboratory Press, Cold Spring Harbor, NY, 1988) using an HA specific monoclonal antibody. Briefly, the cells are labeled for 8 hours with ³⁵S-me+ioine (or ³⁵S-cysteine). The culture media are then collected and the cells are lysed using detergents (RIPA buffer, 150 mM NaCl, 1% NP-40, 0.1 % SDS, 0.5% DOC, 50 mM Tris, pH 7.5). Both the cell lysate and the culture media are precipitated with an HA specific monoclonal antibody. Precipitated polypeptides are then analyzed by SDS-PAGE.

Alternatively, DNA containing the 18480 coding sequence is cloned directly into the polylinker of the pCDNA/Amp vector using the appropriate restriction sites. The resulting plasmid is transfected into COS cells in the manner described above, and the expression of the 18480 polypeptide is detected by radiolabelling and immunoprecipitation using a 18480 specific monoclonal antibody.

## Claims

1. An isolated nucleic acid molecule selected from the group consisting of:
a) a nucleic acid molecule comprising a fragment of at least 500 nucleotides of the nucleotide sequence of SEQ ID NO:4 or SEQ ID NO:6;
b) a nucleic acid molecule which encodes a polypeptide comprising the amino acid sequence of SEQ ID NO:5; and
c) a nucleic acid molecule comprising the nucleotide sequence of SEQ ID NO:4 or SEQ ID NO:6.

2. The isolated nucleic acid molecule of claim 1, which is the nucleotide sequence SEQ ID NO:4.

3. A host cell which contains the nucleic acid molecule of claim 1.

4. An isolated polypeptide selected from the group consisting of:
a) a polypeptide comprising an amino acid sequence which is at least 80% identical to the amino acid sequence of a polypeptide as shown in SEQ ID NO:5; and
b) the amino acid sequence of SEQ ID NO:5.

5. The isolated polypeptide of claim 4, wherein the polypeptide comprises an amino acid sequence which is at least 85%, 90%, 95% or 98% identical to the amino acid sequence of a polypeptide as shown in SEQ ID NO:5

6. The polypeptide of claim 4 wherein the polypeptide is linked to a polypeptide having an amino acid sequence which is different from the polypeptide of claim 5.

7. An antibody or antigen-binding portion thereof, which selectively binds to a polypeptide of claim 4.

8. The antibody or antigen-binding portion thereof of claim 7, wherein the antibody is detectably labeled.

9. The antibody or antigen-binding portion thereof of claim 8, wherein the detectable label is selected from the group consisting of:
a) enzymes;
b) prosthetic groups;
c) fluorescent materials;
d) luminescent materials;
e) bio luminescent materials; and
f) radioactive materials.

10. The antibody or antigen-binding portion thereof of claim 7, wherein the antibody is selected from the group consisting of:
a) monoclonal antibody;
b) polyclonal antibody;
c) Fab fragment;
d) F(ab')₂ fragment;
e) chimeric antibody;
f) humanized antibody; and
g) human antibody

11. A method for producing a polypeptide selected from the group consisting of:
a) a polypeptide comprising the amino acid sequence of SEQ ID NO:5;
b) a polypeptide comprising a fragment of the amino acid sequence of SEQ ID NO:5, wherein the fragment comprises at least 200 contiguous amino acids of SEQ ID NO:5; and
c) the amino acid sequence of SEQ ID NO: 5; comprising culturing the host cell of claim 3 under conditions in which the nucleic acid molecule is expressed.

12. A method for detecting the presence of a nucleic acid molecule of claim 1 or a polypeptide encoded by the nucleic acid molecule in a sample, comprising:
a) contacting the sample with a compound which selectively hybridizes to the nucleic acid molecule of claim 1 or an antibody which binds to the polypeptide encoded by the nucleic acid molecule; and
b) determining whether the compound hybridizes to the nucleic acid or the antibody binds to the polypeptide in the sample.

13. A kit comprising an antibody which selectively binds to a polypeptide of claim 4 and instructions for use.

14. A method for identifying a compound which binds to a polypeptide or modulates the activity of the polypeptide of claim 4 comprising the steps of:
a) contacting a polypeptide, or a cell expressing a polypeptide of claim 4 with a test compound; and
b) determining whether the polypeptide binds to the test compound or determining the effect of the test compound on the activity of the polypeptide.

15. A method for modulating the activity of a polypeptide of claim 4 comprising contacting the polypeptide or a cell expressing the polypeptide with an antibody which binds to the polypeptide in a sufficient concentration to modulate the activity of the polypeptide.

16. A method of identifying a subject having a lung cancer or a colon cancer or at risk for developing a lung cancer or a colon cancer, comprising:
a) contacting a first sample obtained from the subject comprising polypeptides with an antibody which binds to the polypeptide defined in claim 4;
b) detecting the presence of a polypeptide in the sample that binds to the antibody;
c) contacting a second sample obtained from a control subject comprising polypeptides with an antibody which binds to the polypeptide defined in claim 4;
d) detecting the presence of a polypeptide in the sample that binds to the antibody; and
e) comparing the level of antibody binding in the first sample relative to the level of antibody binding in the second sample,
wherein an increase in the level of antibody binding in the first sample relative to the level of antibody binding in the second sample identifies a subject having a lung cancer or a colon cancer, or at risk for developing a lung cancer or a colon cancer.

17. A method for identifying a compound capable of treating a lung cancer or a colon cancer, **characterized by** aberrant expression of a nucleic acid of claim 1 or aberrant activity of a polypeptide of claim 4 comprising assaying the ability of the compound to modulate expression of a nucleic acid of claim 1 or activity of a polypeptide of claim 4, thereby identifying a compound capable of treating a lung cancer or a colon cancer.

## Patentansprüche

1. Ein isoliertes Nukleinsäuremolekül, ausgewählt aus der Gruppe bestehend aus:
a) einem Nukleinsäuremolekül umfassend ein Fragment von mindestens 500 Nukleotiden der Nukleotidsequenz der SEQ ID NO:4 oder SEQ ID NO:6;
b) einem Nukleinsäuremolekül, das ein die Aminosäuresequenz der SEQ ID NO:5 umfassendes Polypeptid kodiert; und
c) einem Nukleinsäuremolekül umfassen die Nukleotidsequenz der SEQ ID NO:4 oder SEQ ID NO:6.

2. Das isolierte Nukleinsäuremolekül von Anspruch 1, das die Nukleotidsequenz SEQ ID NO:4 ist.

3. Eine Wirtszelle, die das Nukleinsäuremolekül von Anspruch 1 enthält.

4. Ein isoliertes Polypeptid, ausgewählt aus der Gruppe bestehend aus:
a) einem Polypeptid umfassend eine Aminosäuresequenz, die zu mindestens 80% mit der Aminosäuresequenz eines wie in SEQ ID NO:5 gezeigten Polypeptids identisch ist; und
b) der Aminosäuresequenz der SEQ ID NO:5.

5. Das isolierte Polypeptid von Anspruch 4, wobei das Polypeptid eine Aminosäuresequenz umfasst, die zu mindestens 85%, 90%, 95% oder 98% identisch ist mit der Aminosäuresequenz eines wie in SEQ ID NO:5 gezeigten Polypeptids.

6. Das Polypeptid von Anspruch 4, wobei das Polypeptid an ein Polypeptid gebunden ist, das eine Aminosäuresequenz hat, die von dem Polypeptid des Anspruchs 5 verschieden ist.

7. Ein Antikörper oder ein Antigen-bindender Teil davon, der selektiv an das Polypeptid von Anspruch 4 bindet.

8. Der Antikörper oder der Antigen-bindende Teil davon von Anspruch 7, wobei der Antikörper mit einer nachweisbaren Markierung versehen ist.

9. Der Antikörper oder der Antigen-bindende Teil davon von Anspruch 8, wobei die nachweisbare Markierung ausgewählt ist aus der Gruppe bestehend aus:
a) Enzyme;
b) prosthetische Gruppen;
c) fluoreszierende Materialien;
d) lumineszierende Materialien;
e) biolumineszierende Materialien; und
f) radioaktive Materialien.

10. Der Antikörper oder der Antigen-bindende Teil davon von Anspruch 7, wobei der Antikörper ausgewählt ist aus der Gruppe bestehend aus:
a) monoklonaler Antikörper;
b) polyklonaler Antikörper;
c) Fab-Fragment;
d) F(ab')₂-Fragment;
e) chimerer Antikörper;
f) humanisierter Antikörper; und
g) humaner Antikörper.

11. Ein Verfahren zur Herstellung eines Polypeptids ausgewählt aus der Gruppe bestehend aus:
a) einem Polypeptid umfassend die Aminosäuresequenz der SEQ ID NO:5;
b) einem Polypeptid umfassend ein Fragment der Aminosäuresequenz der SEQ ID NO:5, wobei das Fragment mindestens 200 zusammenhängende Aminosäuren der SEQ ID NO:5 umfasst; und
c) der Aminosäuresequenz der SEQ ID NO:5; umfassend die Kultivierung der Wirtszellen von Anspruch 3 unter Bedingungen, bei denen das Nukleinsäuremolekül exprimiert wird.

12. Ein Verfahren zur Detektion der Anwesenheit eines Nukleinsäuremoleküls von Anspruch 1 oder eines von dem Nukleinsäuremolekül kodierten Polypeptids in einer Probe, umfassend:
a) in Kontakt bringen der Probe mit einer Verbindung, die selektiv mit dem Nukleinsäuremolekül von Anspruch 1 hybridisiert, oder mit einem Antikörper, der an das von dem Nukleinsäuremolekül kodierten Polypeptid bindet; und
b) bestimmen, ob die Verbindung mit der Nukleinsäure hybridisiert oder ob der Antikörper an das Polypeptid in der Probe bindet.

13. Ein Kit umfassend einen Antikörper, der selektiv an ein Polypeptid von Anspruch 4 bindet, und eine Gebrauchsanweisung.

14. Ein Verfahren zur Identifizierung einer Verbindung, die an ein Polypeptid bindet oder die Aktivität des Polypeptids von Anspruch 4 moduliert, umfassend die Schritte:
a) in Kontakt bringen eines Polypeptids, oder einer ein Polypeptid von Anspruch 4 exprimierenden Zelle mit einer Testverbindung; und
b) bestimmen, ob das Polypeptid an die Testverbindung bindet, oder bestimmen des Effekts der Testverbindung auf die Aktivität des Polpeptids.

15. Ein Verfahren zur Modulation der Aktivität eines Polypeptids von Anspruch 4, umfassend das in Kontakt bringen des Polypeptids, oder einer das Polypeptid exprimierenden Zelle mit einem Antikörper, der an das Polypeptid bindet, in einer ausreichenden Konzentration um die Aktivität des Polypeptids zu modulieren.

16. Ein Verfahren zur Identifikation in einem Subjekt mit Lungenkrebs oder Darmkrebs oder mit dem Risiko, Lungenkrebs oder Darmkrebs zu entwickeln, umfassend:
a) in Kontakt bringen einer ersten von dem Subjekt erhaltenen, Polypeptide umfassenden Probe mit einem Antikörper, der an das in Anspruch 4 definierte Polypeptid bindet;
b) Detektion der Anwesenheit eines Polypeptids in der Probe, das an den Antikörper bindet;
c) in Kontakt bringen einer zweiten, von einem Kontrollsubjekt erhaltenen, Polypeptide umfassenden Probe mit einem Antikörper, der an das in Anspruch 4 definierte Polypeptid bindet;
d) Detektion der Anwesenheit eines Polypeptids in der Probe, das an den Antikörper bindet; und
e) vergleichen des Levels von Antikörper, der in der ersten Probe bindet, mit dem Level von Antikörper, der in der zweiten Probe bindet, wobei ein Anstieg im Level der Antikörperbindung in der ersten Probe im Vergleich zum Level der Antikörperbindung in der zweiten Probe ein Subjekt identifiziert, das Lungenkrebs oder Darmkrebs hat oder ein Risiko, Lungenkrebs oder Darmkrebs zu entwickeln.

17. Ein Verfahren zur Identifikation einer Verbindung, die zur Behandlung von Lungenkrebs oder Darmkrebs, welcher durch die abweichende Expression eines Nukleinsäuremoleküls von Anspruch 1 oder eine abweichende Aktivität eines Polypeptids von Anspruch 4 **gekennzeichnet** ist, eingesetzt werden kann, umfassend die Untersuchung der Fähigkeit der Verbindung, die Expression der Nukleinsäure von Anspruch 1 oder die Aktivität eines Polypeptids von Anspruch 4 zu modulieren, und **dadurch** die Identifizierung eine Verbindung, die zur Behandlung von Lungenkrebs oder Darmkrebs eingesetzt werden kann.

## Revendications

1. Molécule d'acide nucléique isolée choisie dans le groupe constitué par :
a) une molécule d'acide nucléique comprenant un fragment d'au moins 500 nucléotides de la séquence nucléotidique de SEQ ID NO: 4 ou SEQ ID NO:6 ;
b) une molécule d'acide nucléique qui code pour un polypeptide comprenant la séquence d'acides aminés de SEQ ID NO: 5 ;
et
c) une molécule d'acide nucléique comprenant la séquence nucléotidique de SEQ ID NO:4 ou SEQ ID NO:6.

2. Molécule d'acide nucléique isolée selon la revendication 1, qui est la séquence nucléotidique SEQ ID NO:4.

3. Cellule hôte qui contient la molécule d'acide nucléique de la revendication 1.

4. Polypeptide isolé choisi dans le groupe constitué par :
a) un polypeptide comprenant une séquence d'acides aminés qui est à au moins 80 % identique à la séquence d'acides aminés d'un polypeptide tel que présenté dans SEQ ID NO:5 ;
et
b) la séquence d'acides aminés de SEQ ID NO:5.

5. Polypeptide isolé selon la revendication 4, dans lequel le polypeptide comprend une séquence d'acides aminés qui est à au moins 85 %, 90 %, 95 % ou 98 % identique à la séquence d'acides aminés d'un polypeptide tel que présenté dans SEQ ID NO: 5.

6. Polypeptide selon la revendication 4, dans lequel le polypeptide est lié à un polypeptide ayant une séquence d'acides aminés qui est différente du polypeptide de la revendication 5.

7. Anticorps ou partie de liaison à un antigène de celui-ci, qui se lie de façon sélective à un polypeptide de la revendication 4.

8. Anticorps ou partie de liaison à un antigène de celui-ci selon la revendication 7, dans lequel l'anticorps est marqué de façon détectable.

9. Anticorps ou partie de liaison à un antigène de celui-ci selon la revendication 8, dans lequel le marqueur détectable est choisi dans le groupe constitué par :
a) les enzymes ;
b) les groupes prosthétiques ;
c) les matières fluorescentes ;
d) les matières luminescentes ;
e) les matières bioluminescentes ; et
f) les matières radioactives.

10. Anticorps ou partie de liaison à un antigène de celui-ci selon la revendication 7, dans lequel l'anticorps est choisi dans le groupe constitué par :
a) un anticorps monoclonal ;
b) un anticorps polyclonal ;
c) un fragment Fab ;
d) un fragment F(ab')₂ ;
e) un anticorps chimérique ;
f) un anticorps humanisé ; et
g) un anticorps humain.

11. Procédé de fabrication d'un polypeptide choisi dans le groupe constitué par :
a) un polypeptide comprenant la séquence d'acides aminés de SEQ ID NO:5 ;
b) un polypeptide comprenant un fragment de la séquence d'acides aminés de SEQ ID NO:5, le fragment comprenant au moins 200 acides aminés contigus de SEQ ID NO:5 ; et
c) la séquence d'acides aminés de SEQ ID NO:5 ;
comprenant la culture de la cellule hôte de la revendication 3 dans des conditions dans lesquelles la molécule d'acide nucléique est exprimée.

12. Procédé de détection de la présence dans un échantillon d'une molécule d'acide nucléique de la revendication 1 ou d'un polypeptide codé par la molécule d'acide nucléique, comprenant les opérations consistant à :
a) mettre en contact l'échantillon avec un composé qui s'hybride de façon sélective à la molécule d'acide nucléique de la revendication 1 ou un anticorps qui se lie au polypeptide codé par la molécule d'acide nucléique ; et
b) déterminer si ou non le composé s'hybride à l'acide nucléique ou l'anticorps se lie au polypeptide dans l'échantillon.

13. Coffret comprenant un anticorps qui se lie de façon sélective à un polypeptide de la revendication 4 et des instructions d'utilisation.

14. Procédé d'identification d'un composé qui se lie à un polypeptide ou module l'activité du polypeptide de la revendication 4 comprenant les étapes consistant à :
a) mettre en contact un polypeptide ou une cellule exprimant un polypeptide de la revendication 4 avec le composé d'essai ; et
b) déterminer si ou non le polypeptide se lie au composé d'essai ou déterminer l'effet du composé d'essai sur l'activité du polypeptide.

15. Procédé de modulation de l'activité d'un polypeptide de la revendication 4, comprenant la mise en contact du polypeptide ou d'une cellule exprimant le polypeptide avec un anticorps qui se lie au polypeptide dans une concentration suffisante pour moduler l'activité du polypeptide.

16. Procédé d'identification d'un sujet ayant un cancer du poumon ou un cancer du côlon ou présentant un risque de développer un cancer du poumon ou un cancer du côlon, comprenant les étapes consistant à:
a) mettre en contact un premier échantillon obtenu à partir du sujet comprenant des polypeptides avec un anticorps qui se lie au polypeptide défini dans la revendication 4 ;
b) détecter la présence dans l'échantillon d'un polypeptide qui se lie à l'anticorps ;
c) mettre en contact un second échantillon obtenu à partir d'un sujet témoin comprenant des polypeptides avec un anticorps qui se lie au polypeptide défini dans la revendication 4 ;
d) détecter la présence dans l'échantillon d'un polypeptide qui se lie à l'anticorps ; et
e) comparer le taux de liaison d'anticorps dans le premier échantillon par rapport au taux de liaison d'anticorps dans le second échantillon,
dans lequel une augmentation du taux de liaison d'anticorps dans le premier échantillon par rapport au taux de liaison d'anticorps dans le second échantillon permet d'identifier un sujet ayant un cancer du poumon ou un cancer du côlon, ou à risque de développer un cancer du poumon ou un cancer du côlon.

17. Procédé d'identification d'un composé capable de traiter un cancer du poumon ou un cancer du côlon, **caractérisé par** l'expression aberrante d'un acide nucléique de la revendication 1 ou l'activité aberrante d'un polypeptide de la revendication 4, comprenant le dosage de l'aptitude du composé à moduler l'expression d'un acide nucléique de la revendication 1 ou l'activité d'un polypeptide de la revendication 4, permettant ainsi d'identifier un composé capable de traiter un cancer du poumon ou un cancer du côlon.
